# EUROPEAN PATENT APPLICATION

(11) **EP 4 667 573 A1**
(43) Date of publication of application: **24.12.2025**
(21) Application number: 25765461.6
(22) Date of filing: 24.04.2025
(51) Int. Cl.: C12N 15/67, C12N 15/861, A61K 48/00, A61P 27/02, C12N 15/113

(54) **USE OF MULTIPLE PROMOTERS EFFICIENTLY TARGETING ON-TYPE BIPOLAR CELLS IN GENE THERAPY TECHNOLOGY**

(30) Priority: 23.09.2024 CN 202411328903
(71) Applicant: ZHONGMOU THERAPEUTICS CO., LTD., Wuhan, Hubei 430074 (CN)
(72) Inventor: SHEN, Yin, Wuhan, Hubei 430074 (CN)
(74) Representative: Lavoix
(86) International application number: PCT/CN2025/090797
(87) International publication number: WO 2025/201567

(57) **Abstract**

The present application relates to the fields of biotechnology and gene therapy technology. The present application relates to retinal ON-bipolar cell-specific promoter sequences and use thereof in gene therapy technology to regulate the expression of therapeutic genes in the retina or retinal organoid cells to improve and/or restore vision. Retina bipolar cells are divided into ON-bipolar cells (ON-bipolar cells or depolarized bipolar cells) and OFF-bipolar cells (OFF-bipolar cells or hyperpolarized bipolar cells). The differential gene expression characteristics of different types of bipolar cells are utilized to achieve precise and specific expression of transgenes, thereby achieving efficient and precise treatment of retinal diseases. The advantage of the present application is the utilization of the promoter of the transient receptor potential cation channel protein (TRPM1) gene, which enables high level and highly specific expression in ON-bipolar cells.

## Description

### TECHNICAL FIELD

The present application relates to the field of biotechnology, and in particular to use of multiple enhancer or promoter elements capable of regulating the specific expression of genes in retinal bipolar cells in human ophthalmology retinal related diseases, retinal macular region-related diseases, especially retinitis pigmentosa, to drive the expression of therapeutic transgenes in retinal bipolar cells to achieve the purpose of restoring and/or improving vision.

### BACKGROUND ART

Inherited retinal diseases (IRDs) have an incidence of 1/5000 to 1/3000 and are currently one of the most common causes of blindness (PMID: 36362249). Retinal diseases such as retinitis pigmentosa (RP), Stargardt disease, and age-related macular degeneration (AMD) are characterized by progressive photoreceptor cell death, leading to partial or complete blindness (PMID: 33686777). Conventional therapies for RP, Stargardt disease, and AMD have limited efficacy and fail to prevent photoreceptor cell apoptosis and subsequent blindness. Gene therapy technology refers to the delivery of therapeutic nucleic acid molecules (DNA or RNA), either endogenous or exogenous, for replacing or silencing defective genes, with the aim to slowing disease progression, alleviating symptoms, or supplementing lost function. In the retinas of patients with RP, Stargardt disease, and AMD, although photoreceptor cells undergo degeneration, the residual retinal structures are capable of maintaining both structural and functional integrity for a limited period of time. Optogenetic therapies have been shown to effectively improve vision in RP model mice by expressing photosensitive proteins in residual retinal cells, thereby inducing light responsiveness in cells that were previously non-responsive to light. Some disclosed clinical trials have also shown that optogenetic technology can improve vision in RP patients. For example, an ongoing optogenetic therapy clinical trial (NCT04945772) utilizes the expression of the multi-characteristic opsin (vMCO) specifically in retinal ON-bipolar cells (ON-BCs) to restore light sensitivity in the retina. Phase 2b results of this clinical trial indicate that this therapy demonstrates an improvement in vision, revealing that the strategy of expressing light-activated channel proteins in the retina to improve vision in patients with retinitis pigmentosa is feasible under certain conditions in clinical practice.

Retinal bipolar cells are divided into ON-bipolar cells (ON-BCs) and OFF-bipolar cells (OFF-BCs). In the normal retina, upon exposure to light, the photoreceptor cells connected to the presynaptic terminals of ON-BCs reduce glutamate release, which relieves the inhibition of the mGluR6-TRPM1 channel that was previously suppressed by glutamate. This leads to cation influx, depolarizing the ON-BC and activating it. Meanwhile, OFF-BCs are activated by light exposure due to the reduced glutamate signaling from photoreceptor cells, which closes the cation channels and produces a hyperpolarizing response. Therefore, gene therapy technologies that specifically target depolarizing photosensitive proteins in ON-bipolar cells hold great promise for improving visual acuity in patients with retinitis pigmentosa. The ON-BC promoter that has been frequently used in previous research reports is an approximately 200-bp enhancer sequence upstream of the mouse GRM6 gene. The artificial promoter composed of this sequence and the SV40mini promoter can be specifically expressed in bipolar cells. However, this promoter was later shown to have reduced activity in the retina of *rd1* model mice, affecting its clinical application in disease treatment.

Therefore, there is an urgent need to provide a bipolar cell-specific expression regulatory element distinct from the GRM6 gene promoter, to enable its therapeutic application in treating inherited retinal diseases.

### SUMMARY OF THE INVENTION

The objective of the present application is to improve the targeting specificity of existing ophthalmic gene therapy technologies by providing a novel series of hTRPM1 promoters that have high specificity and high expression levels for ON-bipolar cells, as well as their application in the preparation of therapeutic agents for for retinal degenerative diseases. The innovation of this application: Unlike most existing technologies that are based on the modification of promoters or regulatory sequences of the *GRM6* gene, this application is based on a novel promoter or DNA regulatory sequence of the *TRPM1* gene, which is a promoter composed of a completely new, unreported DNA sequence. The promoter or DNA regulatory sequence involved in this application has expression specificity and sufficient expression activity in retinal ON-bipolar cells, and can be efficiently expressed in human retinal organoids, and has the potential to be used to treat human ophthalmology retinal related diseases, especially thoses retinal macular region-related diseases.

The purpose of this application is to provide a variety of new artificially synthesized ON-bipolar cell-specific promoters.

To achieve the above objectives, this application adopts the following technical solutions:
Item 1. An enhancer or enhancer variant, wherein the enhancer comprises the nucleotide sequence as shown in SEQ ID NO:4, SEQ ID NO:35, SEQ ID NO:6, SEQ ID NO:37, SEQ ID NO:9, SEQ ID NO:10 or SEQ ID NO:39, or the nucleotide sequence truncated from the 5'-end or 3'-end of the nucleotide sequence as shown in SEQ ID NO:4, SEQ ID NO:35, SEQ ID NO:6, SEQ ID NO:37, SEQ ID NO:9, SEQ ID NO:10 or SEQ ID NO:39 to 40-910 base pairs in length, preferably to 56-880 base pairs in length;
   wherein the enhancer variant is at least 60%, 65%, 70%, 75%, 80%, 85%, 90%, 95%, 96%, 97%, 98%, or 99% identical to the enhancer.
Item 2. The enhancer or enhancer variant according to item 1, wherein the enhancer comprises the nucleotide sequence as shown in any one of SEQ ID NOs: 4-14 and 35-39, or a nucleotide sequence having at least 60%, 65%, 70%, 75%, 80%, 85%, 90%, 95%, 96%, 97%, 98%, or 99% sequence identity thereto.
Item 3. A promoter or promoter variant, wherein the promoter comprises the nucleotide sequence as shown in SEQ ID NO: 1 or SEQ ID NO: 33, or the nucleotide sequence truncated from the 5'-end or 3'-end of the nucleotide sequence as shown in SEQ ID NO: 1 or SEQ ID NO: 33 to 500-800 base pairs in length, preferably to 550-750 base pairs in length;
   wherein the promoter variant is at least 60%, 65%, 70%, 75%, 80%, 85%, 90%, 95%, 96%, 97%, 98%, or 99% identical to the promoter.
Item 4. The promoter or promoter variant according to item 3, wherein the promoter comprises the nucleotide sequence as shown in any one of SEQ ID NOs: 1-3 and 33-34, or a nucleotide sequence having at least 60%, 65%, 70%, 75%, 80%, 85%, 90%, 95%, 96%, 97%, 98%, or 99% sequence identity to any one of SEQ ID NOs: 1-3 and 33-34.
Item 5. An isolated nucleic acid molecule, comprising an enhancer and a promoter, wherein:
   the enhancer is the enhancer according to item 1 or 2; and/or
   the promoter is the promoter according to item 3 or 4.
Item 6. An isolated nucleic acid molecule according to item 5, wherein the isolated nucleic acid molecule consists of one or more of the enhancers or enhancer variants, one or more of the promoters or promoter variants, and optionally a spacer separating the enhancer or enhancer variant from the promoter or promoter variant.
Item 7. The isolated nucleic acid molecule according to item 6, wherein the isolated nucleic acid molecule comprises or consists of a sequence selected from the group consisting of SEQ ID NOs: 15-31 and 40-42, or comprises or consists of a sequence having at least 60% identity to the sequence selected from the group consisting of SEQ ID NOs: 15-31 and 40-42.
Item 8. A nucleic acid expression vector, comprising the isolated nucleic acid molecule according to any one of items 5 to 7.
Item 9. The nucleic acid expression vector according to item 8, wherein the nucleic acid expression vector is a recombinant adeno-associated virus vector (AAV), in particular wherein the nucleic acid expression vector is a recombinant AAV1, AAV2, AAV3, AAV4, AAV5, AAV6, AAV7, AAV8, AAV9, AAV10, AAV11 or AAV12 vector, or a derivative variant of the above recombinant vector.
Item 10. The nucleic acid expression vector according to any one of items 8 to 9, further comprising:
   a transgene, wherein the transgene is a photosensitive protein gene, in particular a photosensitive protein gene comprising or consisting of SEQ ID NO: 46 or 47, and
   b. a sequence encoding capsid protein.
Item 11. An adeno-associated virus particle, virus-like particle, or non-viral particle, comprising the isolated nucleic acid molecule according to any one of items 5 to 7 or the nucleic acid expression vector according to any one of items 8 to 10.
Item 12. A pharmaceutical agent, wherein the pharmaceutical agent is selected from the group consisting of the isolated nucleic acid molecule according to any one of items 5 to 7, the nucleic acid expression vector according to any one of items 8 to 10 and the adeno-associated virus particle, virus-like particle, or non-viral particle according to item 11, and used as a medicament.
Item 13. A pharmaceutical agent, wherein the pharmaceutical agent is selected from the group consisting of the isolated nucleic acid molecule according to any one of items 5 to 7, the nucleic acid expression vector according to any one of items 8 to 10, and the adeno-associated virus particle, virus-like particle, or non-viral particle according to item 11, and used for treating congenital stationary night blindness (CSBN1) or rod-cone and cone-rod dystrophy, more preferably retinitis pigmentosa, Stargardt diseases and age-related macular degeneration, or other diseases for which the therapeutic effects are achieved by expressing therapeutic genes in bipolar cells.
Item 14. A pharmaceutical agent, wherein the pharmaceutical agent is selected from the group consisting of the isolated nucleic acid molecule according to any one of items 5 to 7, the nucleic acid expression vector according to any one of items 8 to 10, and the adeno-associated virus particle, virus-like particle, or non-viral particle according to item 11, and wherein the pharmaceutical agent is administered by:
   a. intravitreal injection,
   b. subretinal injection,
   c. choroidal injection, or
   d. eye drops.

### BRIEF DESCRIPTION OF THE DRAWINGS

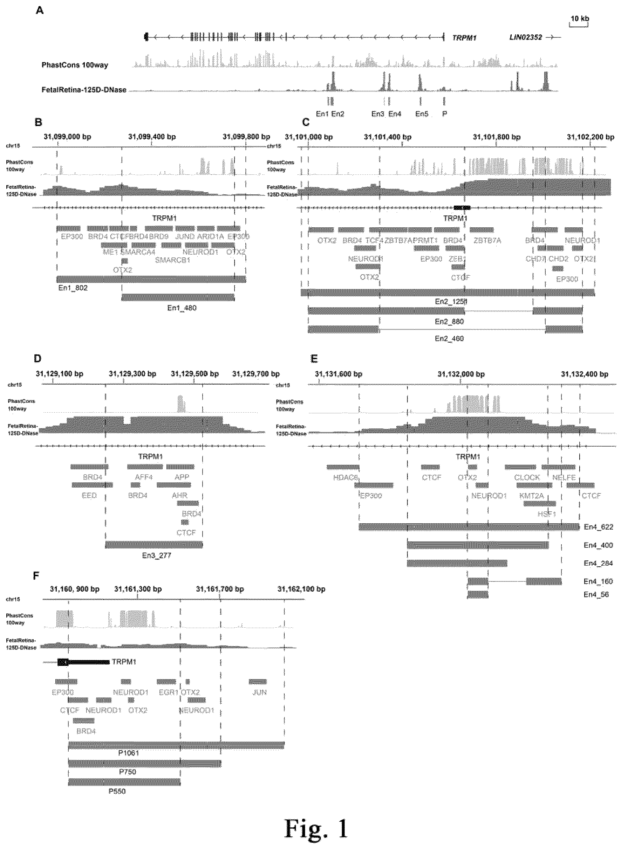

Fig. 1 is a schematic diagram of the localization of enhancer and promoter elements of the human hTRPM1 gene selected in chromatin for promoter design in Example 1. (A) PhastCons 100way is a conserved peak plot across 100 vertebrate genomes. FetalRetina-125D-DNase is a sequencing peak plot of the fetal retinal genome after treatment with DNase I at 125 days of development. Sites with high peaks represent high chromatin accessibility, i.e., strong genomic activity. The gray boxes show that four selected enhancer regions (En1, En2, En3, En4) and one core promoter region (P) have strong chromatin accessibility. All four enhancers are located in the first intron region of the hTRPM1 gene described in Example 1. (B-F) Localization of the (B) En1, (C) En2, (D) En3, (E) En4, and (F) P core promoters and their respective truncated sequences in Figure A in human chromosome 15. The light gray boxes show Chip-seq enriched regions for transcription factors. The dark grey boxes show the positions of truncated sequences, and numbers indicate chromatin position numbers.

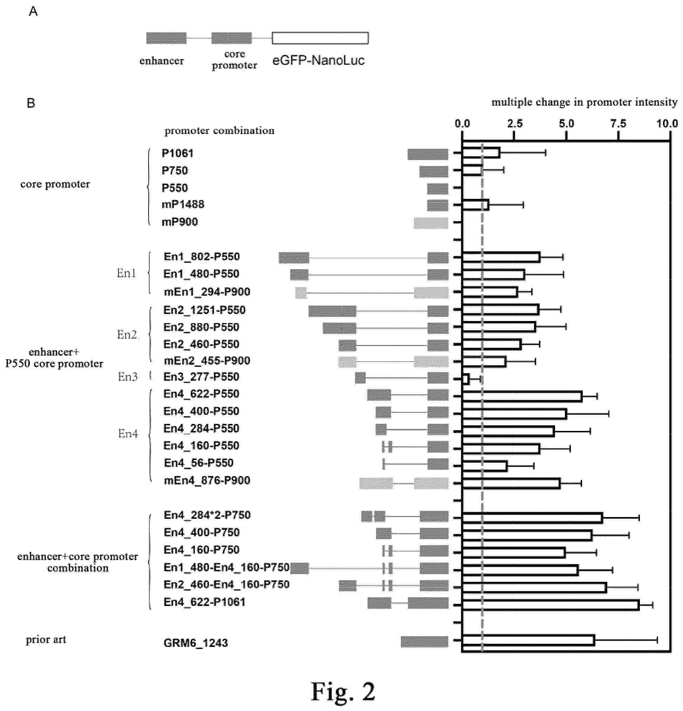

Fig. 2 is a diagram showing the design of test promoter vector elements. (A) The diagram shows that the enhancer in the promoter vector connected to the 5' end of the core promoter and the GFP-NanoLuc fusion reporter gene connected to the 3' end of the core promoter. (B) The diagram shows that the testing combination scheme of the promoter (see Example 2 for details), promoter numbers, and fold change in promoter strength normalized to the NanoLuc expression intensity of the pAAV-P750(hTRPM1)-GFP-NanoLuc-WPRE-hGHpA vector.

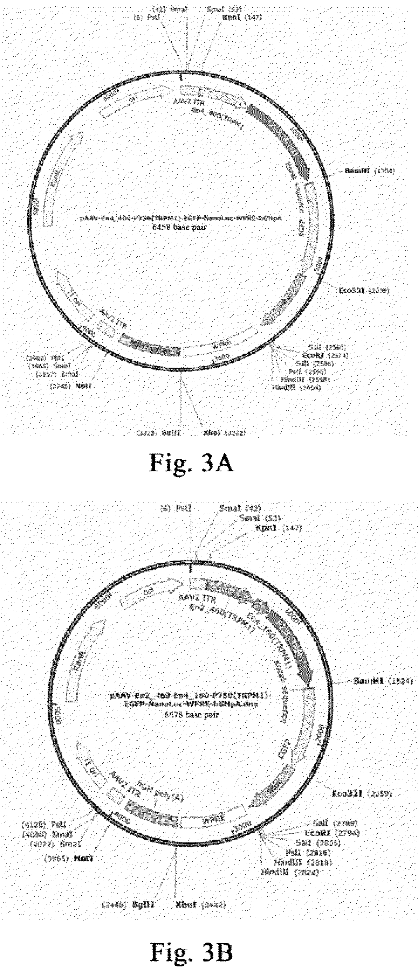

Fig. 3 is a schematic diagram of the test promoter vector patterns used in Examples 1, 2, 3, and 4. The exemplary novel En4_400-P750(TRPM1)(A) and En2_460-En4_160-P750(TRPM1)(B) promoters are inserted into 5' end of GFP and NanoLuc fusion reporter genes. The Kozak sequence is a translation initiation regulatory sequence, while WPRE and hGHpA (i.e., hGH poly(A)) are transcriptional regulatory sequences. The 5' ITR and 3' ITR sequences can mediate transgene packaging within the AAV capsid.

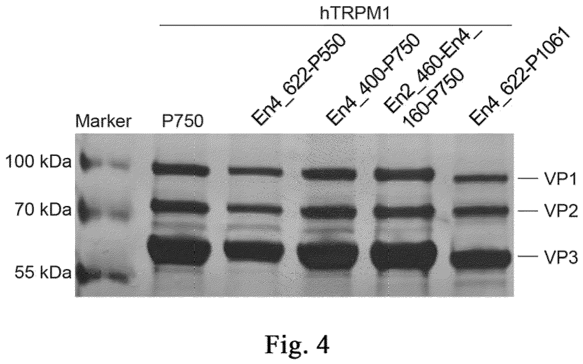

Fig. 4 is an SDS-PAGE silver staining image of the promoter test virus in Examples 2, 3, and 4. The image shows obvious VP1, VP2, and VP3 capsid protein bands of the AAV virus, indicating high virus purity.

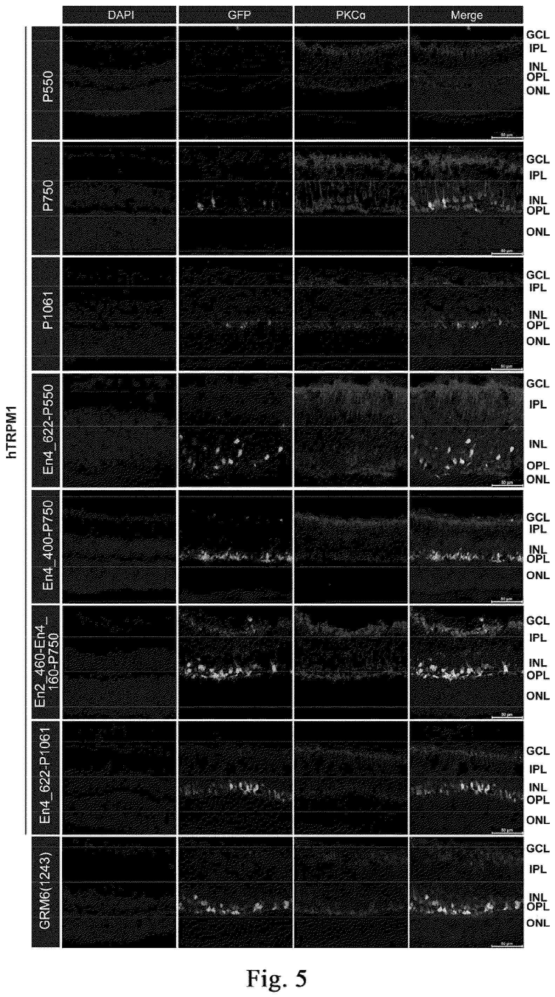

Fig. 5 is an immunofluorescence staining diagram of the mouse retina 3 weeks after intravitreal injection of AAV virus containing different promoters in Example 3 for 3 weeks. The figure shows the expression of green fluorescent protein mediated by P550(hTRPM1), P750(hTRPM1), P1061(hTRPM1), En4_622-P550(hTRPM1), En4_400-P750(hTRPM1), En2_460-En4_160-P750(hTRPM1), En4_622-P1061(hTRPM1) and hGRM6(1243) promoters in the inner nuclear layer (INL) of the retina. The green fluorescence displayed by the green fluorescence channel (GFP channel) indicates the expression of green fluorescent protein (GFP) mediated by the promoter, the red fluorescence displayed by the PKCα channel is the rod bipolar cells recognized by the PKCα antibody, the blue fluorescence displayed by the DAPI channel is the nucleus staining labeled by DAPI, and Merge is the overlap of the fluorescence images displayed by the above three channels. Bar=50µm.

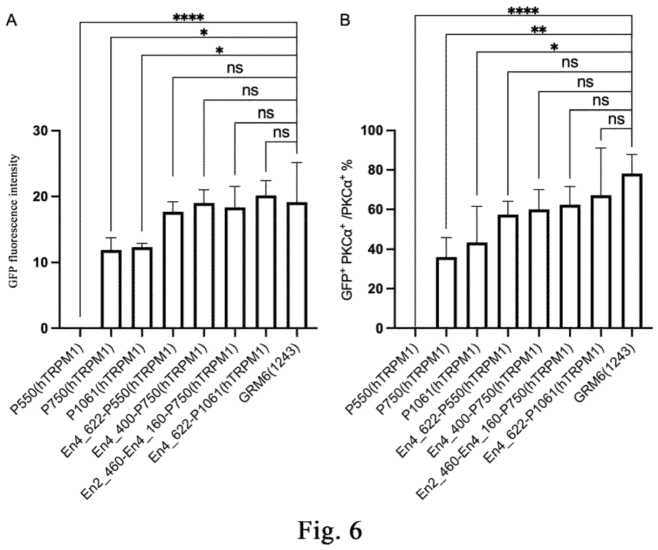

Fig. 6 shows the fluorescence intensity of the hTRPM1 series promoters expressed in (A) the retina and the specificity of their expression in (B) ON-bipolar cells. (A) Statistics of fluorescence signal intensity after image background correction. (B) Statistical analysis of the percentage of cells co-labeled with GFP and PKCα compared to PKCα-labeled cells, indicating the expression specificity of promoters in ON-bipolar cells.

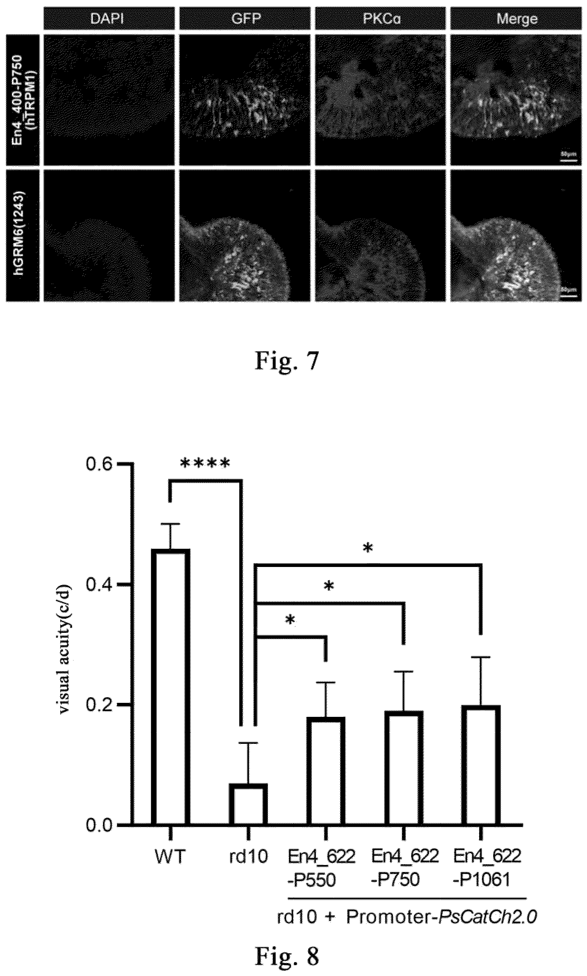

Fig. 7 shows the immunofluorescence staining results of bipolar cells infected with AAV virus containing the hTRPM1 series promoter in human retinal organoids. The green fluorescence displayed by the green fluorescence (GFP) channel indicates promoter-mediated GFP protein expression. The red fluorescence displayed by the PKCα channel indicates rod bipolar cells recognized by the PKCα antibody. The blue fluorescence by the DAPI channel is the staining of DAPI labeled cell nuclei. Merge represents the overlap of the fluorescence images displayed by the three channels. Bar=50 µm.
Fig. 8 shows the visual acuity analysis of *rd10* mice following treatment with an AAV gene therapy containing a promoter-driven photosensitive protein. The exemplary En4_622-P550(hTRPM1)-PsCatCh2.0 and En4_400-P750(hTRPM1)-PsCatCh2.0 significantly improved the visual acuity of *rd10* mice.

### DETAIL DESCRIPTION OF THE INVENTION

The following describes the specific embodiments of the present invention in detail, but it should be understood that these examples are only used to illustrate the present application and are not intended to limit the scope of the present application. Experimental methods in the following examples where specific experimental conditions are not specified are generally performed under conventional conditions or conditions recommended by the manufacturer.

Unless expressly stated otherwise, throughout the specification and claims, the term "include" or variations such as "comprise" or "comprising", etc., will be understood to include the stated compositions or components but not to exclude other compositions or components.

### Term

In the context of this specification, the term "AAV capsid" refers to a synthetic capsid (cap) gene. The AAV capsid disclosed herein can be used to package a recombinant adeno-associated virus for gene therapy.

In the context of this specification, the term "transgene" refers to a gene or genetic substance that has been transferred from one organism to another. In the context herein, the term can also refer to the transfer of a natural or physiologically intact variant of a gene sequence into a patient tissue in which it is absent. It can also refer to the transfer of a native coding sequence whose expression is driven by a promoter that is absent or silent in the target tissue. The term transgene as used herein refers to a polynucleotide encoding a polypeptide of interest that, when expressed in a damaged or diseased retina, can be used to improve or restore vision. Transgenes of particular interest for restoring light sensitivity or vision include photosensitive proteins, such as opsin genes, i.e., channelrhodopsins, vertebrate opsins, and variants thereof.

In the context of this specification, the term "intravitreal administration" or "intravitreal injection" refers to a route of administration of a pharmaceutical agent (e.g., a virus) by which the pharmaceutical agent is delivered to the vitreous body of the eye. Intravitreal administration (or intravitreal injection) is a procedure in which a drug is placed directly into the space at the back of the eye, called the vitreous cavity, which is filled with a jelly-like fluid called vitreous hydrogel.

In the context of this specification, the term "subretinal administration" or "subretinal injection" relates to a route of administering an agent, particularly a virus in the context of this specification, into the space between bipolar cells, photoreceptors and retinal pigment epithelial cells.

In the context of this specification, a broad "promoter" is a nucleic acid sequence that can regulate the transcription level of a gene. The term promoter in this context includes the core promoter sequence adjacent to the gene translation initiation site and also includes enhancer sequences.

In the context of this specification, an "enhancer" is a nucleic acid, a type of non-coding DNA cis-acting element, which can be located upstream, downstream or intronic regions of a gene and has the function of regulating gene transcription levels.

In a first aspect, the present application provides an enhancer or enhancer variant, wherein the enhancer comprises the nucleotide sequence as shown in SEQ ID NO:4, SEQ ID NO:35, SEQ ID NO:6, SEQ ID NO:37, SEQ ID NO:9, SEQ ID NO:10 or SEQ ID NO:39, or the nucleotide sequence truncated from the 5'-end or 3'-end of the nucleotide sequence as shown in SEQ ID NO:4, SEQ ID NO:35, SEQ ID NO:6, SEQ ID NO:37, SEQ ID NO:9, SEQ ID NO:10 or SEQ ID NO:39 to 40-910 base pairs in length, such as 40, 50, 60, 70, 80, 90, 100, 150, 200, 250, 300, 350, 400, 450, 500, 550, 600, 650, 700, 750, 800, 850, 900, or 910 base pairs, etc., preferably to 56-880 base pairs in length, such as 56, 57, 58, 59, 60, 70, 80, 90, 100, 150, 200, 250, 300, 350, 400, 450, 500, 550, 600, 650, 700, 750, 800, 820, 840, 860, or 880 base pairs, or the base pair in any length within the range of 56-880 base pairs in length.

In some embodiments, the enhancer comprises the nucleotide sequence as shown in SEQ ID NO: 4 or the nucleotide sequence truncated from the 5'-end or 3'-end of the nucleotide sequence as shown in SEQ ID NO: 4 to 40-910 base pairs in length, such as 40, 50, 52, 54, 56, 58, 60, 70, 80, 90, 100, 150, 200, 250, 300, 350, 400, 450, 500, 550, 600, 650, 700, 750, 800, 820, 840, 850, 860, 870, 880, 900, or 910 base pairs, etc., preferably to 56-880 base pairs in length, such as 56, 57, 58, 59, 60, 70, 80, 90, 100, 150, 200, 250, 300, 350, 360, 370, 380, 390, 400, 450, 500, 550, 600, 650, 700, 750, 800, 820, 840, 860 or 880 base pairs, or the base pair in any length within the range of 56-880 base pairs in length.

In some embodiments, the enhancer comprises the nucleotide sequence as shown in SEQ ID NO: 35 or the nucleotide sequence truncated from the 5'-end or 3'-end of the nucleotide sequence as shown in SEQ ID NO: 35 to 40-910 base pairs in length, such as 40, 50, 52, 54, 56, 58, 60, 70, 80, 90, 100, 150, 200, 250, 300, 350, 400, 450, 500, 550, 600, 650, 700, 750, 800, 820, 840, 850, 860, 870, 880, 900, or 910 base pairs, etc., preferably to 56-880 base pairs in length, such as 56, 57, 58, 59, 60, 70, 80, 90, 100, 150, 200, 250, 300, 350, 360, 370, 380, 390, 400, 450, 500, 550, 600, 650, 700, 750, 800, 820, 840, 860 or 880 base pairs, or the base pair in any length within the range of 56-880 base pairs in length.

In some embodiments, the enhancer comprises the nucleotide sequence as shown in SEQ ID NO: 6 or the nucleotide sequence truncated from the 5'-end or 3'-end of the nucleotide sequence as shown in SEQ ID NO: 6 to 40-910 base pairs in length, such as 40, 50, 52, 54, 56, 58, 60, 70, 80, 90, 100, 150, 200, 250, 300, 350, 400, 450, 500, 550, 600, 650, 700, 750, 800, 820, 840, 850, 860, 870, 880, 900, or 910 base pairs, etc., preferably to 56-880 base pairs in length, such as 56, 57, 58, 59, 60, 70, 80, 90, 100, 150, 200, 250, 300, 350, 360, 370, 380, 390, 400, 450, 500, 550, 600, 650, 700, 750, 800, 820, 840, 860 or 880 base pairs, or the base pair in any length within the range of 56-880 base pairs in length.

In some embodiments, the enhancer comprises the nucleotide sequence as shown in SEQ ID NO: 37 or the nucleotide sequence truncated from the 5'-end or 3'-end of the nucleotide sequence as shown in SEQ ID NO: 37 to 40-910 base pairs in length, such as 40, 50, 52, 54, 56, 58, 60, 70, 80, 90, 100, 150, 200, 250, 300, 350, 400, 450, 500, 550, 600, 650, 700, 750, 800, 820, 840, 850, 860, 870, 880, 900, or 910 base pairs, etc., preferably to 56-880 base pairs in length, such as 56, 57, 58, 59, 60, 70, 80, 90, 100, 150, 200, 250, 300, 350, 360, 370, 380, 390, 400, 450, 500, 550, 600, 650, 700, 750, 800, 820, 840, 860 or 880 base pairs, or the base pair in any length within the range of 56-880 base pairs in length.

In some embodiments, the enhancer comprises the nucleotide sequence as shown in SEQ ID NO: 9 or the nucleotide sequence truncated from the 5'-end or 3'-end of the nucleotide sequence as shown in SEQ ID NO: 9 to 40-910 base pairs in length, such as 40, 50, 52, 54, 56, 58, 60, 70, 80, 90, 100, 150, 200, 250, 300, 350, 400, 450, 500, 550, 600, 650, 700, 750, 800, 820, 840, 850, 860, 870, 880, 900, or 910 base pairs, etc., preferably to 56-880 base pairs in length, such as 56, 57, 58, 59, 60, 70, 80, 90, 100, 150, 200, 250, 300, 350, 360, 370, 380, 390, 400, 450, 500, 550, 600, 650, 700, 750, 800, 820, 840, 860 or 880 base pairs, or the base pair in any length within the range of 56-880 base pairs in length.

In some embodiments, the enhancer comprises the nucleotide sequence as shown in SEQ ID NO: 10 or the nucleotide sequence truncated from the 5'-end or 3'-end of the nucleotide sequence as shown in SEQ ID NO: 10 to 40-910 base pairs in length, such as 40, 50, 52, 54, 56, 58, 60, 70, 80, 90, 100, 150, 200, 250, 300, 350, 400, 450, 500, 550, 600, 650, 700, 750, 800, 820, 840, 850, 860, 870, 880, 900, or 910 base pairs, etc., preferably to 56-880 base pairs in length, such as 56, 57, 58, 59, 60, 70, 80, 90, 100, 150, 200, 250, 300, 350, 360, 370, 380, 390, 400, 450, 500, 550, 600, 650, 700, 750, 800, 820, 840, 860 or 880 base pairs, or the base pair in any length within the range of 56-880 base pairs in length.

In some embodiments, the enhancer comprises the nucleotide sequence as shown in SEQ ID NO: 39 or the nucleotide sequence truncated from the 5'-end or 3'-end of the nucleotide sequence as shown in SEQ ID NO: 39 to 40-910 base pairs in length, such as 40, 50, 52, 54, 56, 58, 60, 70, 80, 90, 100, 150, 200, 250, 300, 350, 400, 450, 500, 550, 600, 650, 700, 750, 800, 820, 840, 850, 860, 870, 880, 900, or 910 base pairs, etc., preferably to 56-880 base pairs in length, such as 56, 57, 58, 59, 60, 70, 80, 90, 100, 150, 200, 250, 300, 350, 360, 370, 380, 390, 400, 450, 500, 550, 600, 650, 700, 750, 800, 820, 840, 860 or 880 base pairs, or the base pair in any length within the range of 56-880 base pairs in length.

In some embodiments, enhancer variants and their reverse complements are at least 60%, 65%, 70%, 75%, 80%, 85%, 90%, 95%, 96%, 97%, 98%, or 99% identical to the enhancer, and preferably 99% identical to the enhancer.

In some embodiments, the truncated enhancer variants and their reverse complements are at least 60%, 65%, 70%, 75%, 80%, 85%, 90%, 95%, 96%, 97%, 98%, or 99% identical to the truncated enhancer, and preferably 99% identical to the truncated enhancer.

In some embodiments, the enhancer comprises the nucleotide sequence as shown in any one of SEQ ID NOs: 4-14 and 35-39, or its reverse complement, or a nucleotide sequence having at least 60%, 65%, 70%, 75%, 80%, 85%, 90%, 95%, 96%, 97%, 98%, or 99% sequence identity to any one of SEQ ID NOs: 4-14 and 35-39, preferably a nucleotide sequence having at least 99% sequence identity to any one of SEQ ID NOs: 4-14 and 35-39.

A second aspect of the present application provides a promoter or promoter variant, wherein the promoter comprises the nucleotide sequence as shown in SEQ ID NO: 1 or SEQ ID NO: 33, or the nucleotide sequence truncated from the 5'-end or 3'-end of the nucleotide sequence as shown in SEQ ID NO: 1 or SEQ ID NO: 33 to 500-800 base pairs in length, such as 500, 550, 600, 650, 700, 750, or 800, preferably to 550-750 base pairs in length, such as 550, 600, 650, 700, or 750, or the base pair in any length within the range of 550-750 base pairs in length.

In some embodiments, the promoter comprises the nucleotide sequence as shown in SEQ ID NO: 1 or the nucleotide sequence truncated from the 5'-end or 3'-end of the nucleotide sequence as shown in SEQ ID NO: 1 to 500-800 base pairs in length, such as 500, 550, 600, 650, 700, 750, or 800, preferably to 550-750 base pairs in length, such as 550, 600, 650, 700, or 750, or the base pair in any length within the range of 56-880 base pairs in length.

In some embodiments, the promoter comprises the nucleotide sequence as shown in SEQ ID NO: 33 or the nucleotide sequence truncated from the 5'-end or 3'-end of the nucleotide sequence as shown in SEQ ID NO: 33 to 500-800 base pairs in length, such as 500, 550, 600, 650, 700, 750, or 800, preferably to 550-750 base pairs in length, such as 550, 600, 650, 700, or 750, or the base pair in any length within the range of 56-880 base pairs in length.

In some embodiments, the promoter variants and their reverse complements are at least 60%, 65%, 70%, 75%, 80%, 85%, 90%, 95%, 96%, 97%, 98%, or 99% identical to the promoter, preferably 99% identical to the promoter.

In some embodiments, the truncated promoter variants and their reverse complements are at least 60%, 65%, 70%, 75%, 80%, 85%, 90%, 95%, 96%, 97%, 98%, or 99% identical to the truncated promoter, preferably 99% identical to the truncated promoter.

In some embodiments, the promoter comprises the nucleotide sequence as shown in any one of SEQ ID NOs: 1-3 and 33-34, or a nucleotide sequence having at least 60%, 65%, 70%, 75%, 80%, 85%, 90%, 95%, 96%, 97%, 98%, 99% sequence identity to any one of SEQ ID NOs: 1-3 and 33-34, preferably a nucleotide sequence having at least 99% sequence identity to any one of SEQ ID NOs: 1-3 and 33-34.

In some embodiments, the enhancer is located upstream, downstream, or any other position on the same vector as the promoter.

The third aspect of the present application provides an isolated nucleic acid molecule comprising an enhancer and a promoter, wherein the enhancer is the enhancer described above; and/or the promoter is the promoter described above.

In some embodiments, the isolated nucleic acid molecule consists of one or more enhancers or enhancer variants, one or more promoters or promoter variants, and optionally a spacer separating the enhancer or enhancer variant from the promoter or promoter variant.

In some embodiments, the isolated nucleic acid molecule further comprises a spacer sequence having a length of 1 to 1000 base pairs, particularly 1 to 394 base pairs. In some embodiments, the spacer is located between the enhancer and the promoter. In some embodiments, the isolated nucleic acid molecule further comprises a spacer sequence having a length of 1 to 1000 base pairs, particularly 1 to 394 base pairs, and the spacer is located between the enhancer and the promoter.

In some embodiments, the isolated nucleic acid molecule comprises or consists of a sequence selected from the group consisting of SEQ ID NOs: 15-31 and 38-40. In some embodiments, the isolated nucleic acid molecule comprises or consists of a sequence having at least 60%, e.g., 60%, 65%, 70%, 75%, 80%, 85%, 90%, 95%, 96%, 97%, 98%, 99%, 100% identity to a sequence selected from the group consisting of SEQ ID NOs: 15-31 and 38-40.

The fourth aspect of the present application provides a nucleic acid expression vector comprising the isolated nucleic acid molecule described above.

In some embodiments, the nucleic acid expression vector is a recombinant adeno-associated vector (AAV).

In some embodiments, the nucleic acid expression vector is a recombinant AAV1, AAV2, AAV3, AAV4, AAV5, AAV6, AAV7, AAV8, AAV9, AAV10, AAV11 or AAV 12 vector or a derivative variant thereof. Preferably, the vector is a recombinant AAV2 vector.

In some embodiments, the nucleic acid expression vector further comprises:
a transgene, wherein the transgene is a photosensitive protein gene, in particular a photosensitive protein gene comprising or consisting of SEQ ID NO: 46 or 47, and
b. a sequence encoding capsid protein.

From the 5' end to the 3' end, the isolated nucleic acid molecule comprises: first, the enhancer, followed by an optional spacer, and then the promoter. The transgene is located 3' to the promoter. In some embodiments, the transgene is preceded by an optimized Kozak sequence.

In some embodiments, the transgene is a photosensitive protein gene or an opsin gene that restores light detection or vision.

In some embodiments, the photosensitive protein or opsin is selected from the group consisting of channelrhodopsin, melanopsin, rhodopsin, cone opsin, pinealopsin, photopsin, halorhodopsin, bacteriorhodopsin, proteorhodopsin, aequorin, jumping spider opsin, or any functional variant or fragment thereof.

In some embodiments, the opsin is a chimeric protein between opsin and the metabotropic glutamate receptor mGluR6 of the retinal OBC. Preferably, the chimeric protein is Opto-mGluR6.

In some embodiments, the nucleic acid expression vector further comprises a WPRE (Woodchuck Hepatitis Virus Posttranscriptional Regulatory Element) regulatory sequence. WPRE is a DNA sequence that generates a tertiary structure that enhances expression when transcribed. In some embodiments, the nucleic acid expression vector further comprises a polyadenylic acid tail inserted downstream of the transgene. The polyadenylic acid tail promotes translation of the transgene.

The fifth aspect of the present application provides an adeno-associated virus particle, or a virus-like particle, or a non-viral particle, comprising the isolated nucleic acid molecule according to the third aspect or the nucleic acid expression vector according to the fourth aspect.

The sixth aspect of the present application provides a pharmaceutical agent, wherein the pharmaceutical agent is selected from the group consisting of the isolated nucleic acid molecule according to the third aspect, the nucleic acid expression vector according to the fourth aspect and the adeno-associated virus particle, or virus-like particle, or non-viral particle according to the fifth aspect, and used as a medicament.

Another aspect relates to a pharmaceutical agent, wherein the pharmaceutical agent is selected from the group consisting of the isolated nucleic acid molecule according to the third aspect, the nucleic acid expression vector according to the fourth aspect, and the adeno-associated virus particle, or a virus-like particle, or a non-viral particle according to the fifth aspect, and used for treating congenital stationary night blindness or rod-cone and cone-rod dystrophy, in particular retinitis pigmentosa, Stargardt disease, or macular degeneration.

Another aspect relates to a pharmaceutical agent, wherein the pharmaceutical agent is selected from the group consisting of the isolated nucleic acid molecule according to the third aspect, the nucleic acid expression vector according to the fourth aspect and the adeno-associated virus particle, or virus-like particle, or non-viral particle according to the fifth aspect, and wherein the pharmaceutical agent is administered by:
a. intravitreal administration, in particular intravitreal injection,
b. subretinal administration, in particular subretinal injection,
c. choroidal injection, or
d. eye drops.

Another aspect relates to a treating method, comprising administering the pharmaceutical agent of the present application to a patient in need thereof.

### EXAMPLE

### 1. Materials and Methods

### 1.1 Intravitreal injection

Mice were anesthetized with an isoflurane gas anesthesia system (RWD Life Science Co., Ltd.), and then intravitreal injection was performed. After sufficient anesthesia, the ocular surface and the skin around the eye socket were disinfected with 0.5% active iodine. In order to reduce the discomfort caused by intravitreal injection to mice, topical anesthesia on the mouse eyeballs were performed with proparacaine hydrochloride eye drops (Alcaine). The mice were fixed and the eyeballs were exposed. A microsyringe was used with a 32G insulin needle to draw 1.5 µL of rAAV virus (titer of 1E+13 vg/mL), and the needle was inserted 0.5 mm below the corneoscleral limbus on the nasal side of the mouse to complete the intravitreal injection. The intravitreal injection of the other eye was completed in the same way, and a control group with only the injection preparation was set up. After the injection, levofloxacin hydrochloride eye gel (Jeqi) was applied to the mouse eyeball to prevent infection.

### 1.2 NanoLuc luciferase activity assay

One month after intravitreal injection, mice were killed by cervical dislocation, and the eyeballs were removed and the retinas were dissected using ophthalmic scissors in pre-cooled PBS. According to instructions of the Nano-Glo^{®} Luciferase Assay (Promega, N1110), the retina was transferred to a lysis buffer and ultrasonicated. After sonication for 1 minute, the reaction was incubated at room temperature for 10 minutes and then centrifuged to remove the precipitate. The reaction substrate was prepared according to the instructions and mixed with the retinal lysis buffer. After incubation at room temperature for 5 minutes, luciferase activity was measured using a multi-function microplate reader (Tecan Spark).

### 1.3 Immunofluorescence staining

Mouse eyeballs or retinal organoids were removed and fixed in 4% paraformaldehyde at room temperature for 40 minutes. Retinal tissue was placed in a 30% sucrose solution and dehydrated overnight in a 4°C refrigerator. After embedding in Tissue-Tek OCT Compound, the retinas were sectioned into 14 µm thick sections using a frozen tissue slicer (Leica, Germany, model CM 1950) for immunofluorescence staining. The cut retinas were blocked with 4% BSAT solution for 2 hours at room temperature, incubated with primary antibodies overnight in a 4°C refrigerator, and incubated with secondary antibodies of the corresponding species at room temperature for 2 hours. DAPI (Servicebio, Cat. No. G1012) was incubated at room temperature for 5 minutes, followed by anti-fluorescence quenching mounting medium (Servicebio, Cat. No. G1401). Primary antibodies against GFP (chicken, diluted 1:2000 in retinal sections, Abacm, USA, Cat. No. ab13970) and PKCα (a rod bipolar cell marker, goat, diluted 1:500, Santa Cruz Biotechnology, Cat. No. SC-208-G) were used. Secondary antibodies included Alexa 594 (diluted 1:500, Jackson ImmunoResearch, USA, Cat. No. 705-585-147) and Alexa 488 (diluted 1:500, Jackson ImmunoResearch, USA, Cat. No. 715-545-150). Whole-mount images of the retinas were captured using a Leica Thunder slide scanner and a Zeiss laser confocal microscope (LSM880). Cell counts were analyzed using Cellpose 2.0 and Cellprofiler software.

### 1.4 AAV infection of human retinal organoids

300 µl of (DMEM+N2+FBS) medium was added to a 24-well low-adhesion cell culture plate, and the virus was added at a titer of 1E+11 vg/organoid. After mixing well, human retinal organoids differentiated for 200 days were added. The organoid and virus mixture were incubated in a cell culture incubator at 37°C for 2 hours. Each dish was then supplemented with 700 µl of (DMEM+N2+FBS) medium and mixed, and co-cultured in a cell culture incubator at 37°C. After 2 days of co-culture, the cells were washed twice with culture medium and the medium was completely replaced to remove all excess viral particles. The transduced organoids were cultured in (DMEM+N2+FBS) medium until 10 days after transduction, when samples were collected for observation. Retinal organoids were induced from the H9 human embryonic stem cell line (WiCell, USA).

### Example 1. Design of hTRPM1 promoter

The applicant analyzed the single-cell RNA sequencing data published in the literature (PMID: 37388908; PMID: 27565351; PMID: 32555229) to check the expression pattern of TRPM1. TRPM1 is expressed at a high abundance in ON-BCs of the human retina, and no significant expression is seen in other retinal cells such as photoreceptor cells, ganglion cells, horizontal cells, and vertical cells, indicating that the TRPM1 promoter has great potential for mining ON-BC-specific promoters. According to the reports in the literature (PMID: 32555229), the hTRPM1 transcript with Ensembl gene ID ENST00000542188.5 (NCBI gene ID NM_001252020.2) is expressed at high abundance in retinal cells. Given that this promoter is primarily intended for the treatment of human ophthalmic diseases, to ensure its specific and robust activity in retinas with retinitis pigmentosa, we reviewed the previously published TRPM1 expression pattern in the retina of the *rd1* mouse model (PMID: 33665228). These data showed that TRPM1 gene expression was not downregulated by disease progression, indicating that TRPM1 remains active or even more active in this model, making it suitable for gene therapy technology of retinitis pigmentosa-related ophthalmic diseases. Human retinal DNase I sequencing data (PMID: 29233477) were visualized using the IGV genome browser to analyze cell-type specific chromatin access within TRPM1 gene regulatory sequences. PhastCons 100-way (genomic conservation data for 100 vertebrate species) data was downloaded from the UCSC website (https://hgdownload.cse.ucsc.edu/goldenpath/hg38/phastCons 100way/) to examine genomic conservation across 100 vertebrate, including humans and mice. Four enhancer regions (En) with both chromatin accessibility and genomic conservation, and one core promoter region (P) were obtained. The GTRD website was used to view the transcription factor ChIP sequencing peaks of these four enhancer regions (named En1 to En4 according to the order in which the enhancers appear on the chromatin) and the core promoter region. The transcription factor enrichment regions are as shown in Fig. 1. Considering that the AAV capsid can only accommodate a core plasmid of about 4.5kb in length, the length of the promoter sequence should be shortened as much as possible. Based on ChIP-seq enrichment sites of the transcription factor, we made a series of truncation for enhancers and promoters. The specific sequence is shown in Fig. 1. The specific description is as follows:
**1) core promoter sequence:** there are three versions of the core promoter sequence, namely P1061 (hTRPM1), P750 (hTRPM1), and P550 (hTRPM1). As shown in A and F of Fig. 1, the longest promoter sequence, P1061, is located at -1 to -1061 bp upstream of the 5' end of the translation start site (TLSS) of the TRPM1 (ENST00000542188.5) transcript. Based on the sequence length, it is abbreviated as P1061 (SEQ ID NO: 1). This version was truncated at the 5' end to obtain sequences of 750 bp and 550 bp in length, respectively, abbreviated as P750 (SEQ ID NO: 2) and P550 (SEQ ID NO: 3). The murine homologous sequence of P1061 (hTRPM1) is mP1488 (mTRPM1) (SEQ ID NO: 33); the murine homologous sequence of P550 (hTRPM1) is mP528 (mTRPM1) (SEQ ID NO: 34).
**2) the first enhancer sequence:** as shown in A and B of Fig. 1, there are two versions of the first enhancer region: En1_802 (hTRPM1) and En1_480 (hTRPM1), with sequence lengths of 802 bp and 480 bp, respectively (corresponding to SEQ ID NO: 4 and SEQ ID NO: 5, respectively). The murine homologous sequence of En1_802 (hTRPM1) is mEn1_984 (mTRPM1) (SEQ ID NO: 35). The murine homologous sequence of En1_480 (hTRPM1) is mEn1_664 (mTRPM1) (SEQ ID NO: 36).
**3) the second enhancer sequence:** as shown in A and C of Fig. 1, there are three versions of the second enhancer region, namely En2_1251 (hTRPM1), En2_880 (hTRPM1), and En2_460 (hTRPM1), with sequence lengths of 1251bp, 880bp, and 460bp, respectively (corresponding to SEQ ID NO: 6, SEQ ID NO: 7, and SEQ ID NO: 8, respectively). Among them, En2_880 (hTRPM1) is derived from the original 1251 bp version by removing redundant sequences at both ends and removing sequences with relatively low transcription factor enrichment in the central portion. En2_460 (hTRPM1) is further truncated on the basis of En2_880 (hTRPM1), retaining binding sites of retinal-specific transcription factors OTX2 and NEUROD1 and removing epigenetic factor-related binding sites. The murine homologous sequence of En2_1251 (hTRPM1) is mEn2_1187 (mTRPM1) (SEQ ID NO: 37). The murine homologous sequence of En2_460 is mEn2_542 (mTRPM1) (SEQ ID NO: 38).
**4) the third enhancer sequence:** as shown in A and D of Fig. 1, there is one version of the third enhancer sequence, En3_277 (hTRPM1) (SEQ ID NO: 9);
**5) the fourth enhancer sequence:** as shown in A and E of Fig. 1, there are five versions of the fourth enhancer sequence: En4_622 (hTRPM1), En4_400 (hTRPM1), En4_284 (hTRPM1), En4_160 (hTRPM1), and En4_56 (hTRPM1). The corresponding sequence lengths are 622 bp, 400 bp, 284 bp, 160 bp, and 56 bp, respectively. The details are as follows: the longest enhancer sequence is En4_622 (SEQ ID NO: 10), by removing the less accessible chromatin regions at its 5' and 3' ends, the resulting truncation scheme is En4_400 (hTRPM1) (SEQ ID NO: 11). En_284 (hTRPM1) (SEQ ID NO: 12), En4_160 (hTRPM1) (SEQ ID NO: 13), and En4_56 (hTRPM1) (SEQ ID NO: 14) are obtained by further removing regions with lower chromatin accessibility at both the 5' and 3' ends and retaining evolutionarily conserved regions, and considering transcription factor binding sites for different combinations of schemes. The murine homologous sequence of En4_622 (hTRPM1) is mEn4_876 (mTRPM1) (SEQ ID NO: 39).

### Example 2: Packaging of test promoter vector and virus

In order to evaluate the performance of the promoter in this application, the promoter and enhancer sequences designed in Example 1 were combined and fused with the GFP-NanoLuc gene to construct a test promoter vector. The GFP on the reporter vector can be used to indicate the promoter expression position to determine the promoter specificity, and the NanoLuc gene on the reporter vector is used to indicate the promoter expression intensity (A of Fig. 2 and Fig. 3). The specific combinations of the tested plasmids were divided into 4 categories (B of Fig. 2), as described below (the corresponding relationship between promoter number and promoter combination refers to the following description 1 and Fig. 2):
1) test vectors comprising human and murine TRPM1 core promoters, a total of 5 vectors, as shown below:
   pAAV-P1061(hTRPM1)-GFP-NanoLuc-WPRE-hGHpA,
   pAAV-P750(hTRPM1)-GFP-NanoLuc-WPRE-hGHpA,
   pAAV-P550(hTRPM1)-GFP-NanoLuc-WPRE-hGHpA,
   pAAV-mP1488(mTRPM1)-GFP-NanoLuc-WPRE-hGHpA,
   pAAV-mP528(mTRPM1)-GFP-NanoLuc-WPRE-hGHpA.
2) test vectors comprising the human TRPM1 enhancer + basic version of the P550 core promoter and the murine TRPM1 enhancer + basic version of the mP528 core promoter, a total of 14 vectors (the corresponding promoter sequence information is shown in SEQ ID NOs: 15-25 and 40-42), as shown below:
   En1 enhancer:
      pAAV-En1_802-P550(hTRPM1)-GFP-NanoLuc-WPRE-hGHpA,
      pAAV-En1_480-P550(hTRPM1)-GFP-NanoLuc-WPRE-hGHpA,
   En2 enhancer:
      pAAV-En2_1251-P550(hTRPM1)-GFP-NanoLuc-WPRE-hGHpA,
      pAAV-En2_880-P550(hTRPM1)-GFP-NanoLuc-WPRE-hGHpA,
      pAAV-En2_460-P550(hTRPM1)-GFP-NanoLuc-WPRE-hGHpA,
   En3 enhancer:
      pAAV-En3_277-P550(hTRPM1)-GFP-NanoLuc-WPRE-hGHpA,
   En4 enhancer:
      pAAV-En4_622-P550(hTRPM1)-GFP-NanoLuc-WPRE-hGHpA,
      pAAV-En4_400-P550(hTRPM1)-GFP-NanoLuc-WPRE-hGHpA,
      pAAV-En4_284-P550(hTRPM1)-GFP-NanoLuc-WPRE-hGHpA,
      pAAV-En4_160-P550(hTRPM1)-GFP-NanoLuc-WPRE-hGHpA,
      pAAV-En4_56-P550(hTRPM1)-GFP-NanoLuc-WPRE-hGHpA,
   Murine homologous sequences of some of the above enhancers:
      pAAV-mEn1_664-mP528(mTRPM1)-GFP-NanoLuc-WPRE-hGHpA,
      pAAV-mEn2_542-mP528(mTRPM1)-GFP-NanoLuc-WPRE-hGHpA,
      pAAV- mEn4_876-mP528(mTRPM1)-GFP-NanoLuc-WPRE-hGHpA.
3) combination of enhancer + core promoter, a total of 6 vectors (corresponding promoter sequence information is shown in SEQ ID NO: 26-31), as shown below:
   pAAV-En4_284*2-P750 (hTRPM1)-GFP-NanoLuc-WPRE-hGHpA,
   pAAV-En4_400-P750 (hTRPM1)-GFP-NanoLuc-WPRE-hGHpA,
   pAAV-En4_160-P750 (hTRPM1)-GFP-NanoLuc-WPRE-hGHpA,
   pAAV-En1_480-En4_160-P750 (hTRPM1)-GFP-NanoLuc-WPRE-hGHpA,
   pAAV-En2_460-En4_160-P750 (hTRPM1)-GFP-NanoLuc-WPRE-hGHpA,
   pAAV- En4_622-P1061 (hTRPM1)-GFP-NanoLuc-WPRE-hGHpA.
   wherein, the prior art (PMID: 32258214) was used as a positive control: pAAV-hGRM6(1243)-GFP-NanoLuc-WPRE-hGHpA.

The above core expression plasmid vector was packaged into rAAV vector, and the AAV2-NN mutant serotype (PMID: 33616280) with a higher efficiency of infecting retinal cells was used for intravitreal injection. The specific operation was as follows: the genomic DNA of HEK 293 cells or mouse tissues was extracted as a template, and the promoter and enhancer gene fragments of the human or murine hTRPM1 gene were amplified from the human or mouse genome. The promoter sequence was connected to the linearized pAAV core plasmid vector backbone using recombinase (TransGen Biotech, Cat. No,: CU201), and the connection product was transformed into StbI3 chemical competent cells. The colonies with recombinant plasmids were screened, and the positive colonies of the colony PCR were expanded and cultured, and the plasmids were extracted for sequencing verification. The control promoter expression vector pAAV-hGRM6(1243)-GFP-NanoLuc-WPRE-hGHpA was commissioned to Sangon Biotech (Shanghai) Co., Ltd. to construct. The recombinant adeno-associated virus vector AAV was obtained by packaging and purification using the triple--plasmid transfection method of HEK293 cells, as shown in Fig. 4.

### Example 3. Intravitreal injection of rAAV2-NN to detect the expression intensity of hTRPM1 promoter in mouse retina

C57BL/6J WT mice were injected intravitreally at 4 weeks of age and observed for 1 month after administration. The expression intensity of the promoter was confirmed by measuring the activity of NanoLuc luciferase. The detection results are shown in Fig. 2. The specific results are analyzed as follows:

### 3.1 Core promoter test results

The shortest P550 (hTRPM1) promoter shows almost no activity, while the slightly longer P750 (hTRPM1) promoter exhibits basal expression activity. The expression intensity of this promoter was used as a parameter to measure the expression intensity multiples of other promoters relative to the P750 (hTRPM1) promoter. The longest P1061 (hTRPM1) promoter exhibits 1.85 times the expression intensity of the P750 (hTRPM1) promoter. This indicates that the P750 (hTRPM1) promoter has an appropriate sequence length and possesses the characteristics of basal expression intensity, making it suitable for use as a core promoter in subsequent test and clinical trials.

### 3.2 Enhancer test results

According to the description in Example 1 and Fig. 2, to test the enhancer's own activity, we fused the enhancer with P550 (hTRPM1) with almost no expression intensity. The results are as follows:
(1) regarding the En1-related enhancer, the expression intensity of En1_802-P550 (hTRPM1) is 3.78 times that of the basic version of P750, and the expression intensity of the truncated En1_480-P550 (hTRPM1) is 3.05 times that of the basic version of P750. Since the P550 (hTRPM1) promoter alone has almost no expression activity, it can be inferred that En1_802 (hTRPM1) and En1_480 (hTRPM1) have enhancer activity. The murine homologous sequence of En1_480 (hTRPM1) also has enhancer activity, and the expression intensity of mEn1_664-mP528 (mTRPM1) is 2.71 times that of P750. Since the sequence of En1_480 (hTRPM1) is shorter, the activity loss is not much compared with En1_802 (hTRPM1), and it has greater potential for clinical application.
(2) all three versions of En2-related enhancers in different lengths have enhancer activity. The expression intensity of the enhancers after from long to short combinations with the P500 (hTRPM1) promoter relative to the P750 (hTRPM1) promoter is 3.72, 3.56, and 2.87 times, respectively. Among them, the En2_460 (hTRPM1) promoter has the characteristics of high expression activity and a relatively short sequence, and has great potential for clinical application. Its murine homologous sequence of mEn2_542 (mTRPM1) also has similar expression activity, indicating that conserved homologous sequences between species have similar evolutionarily conserved functions.
(3) The enhancer activity of En3_277 (hTRPM1) was weak and was not tested further.
(4) Five truncated sequences in different lengths related to En4 all have strong enhancer activity. The expression intensity of En4_622 (hTRPM1), En4_400 (hTRPM1), En4_284 (hTRPM1), En4_160 (hTRPM1), and En4_56 (hTRPM1) after from long to short combinations with the P500 (hTRPM1) promoter relative to P750 (hTRPM1) is 5.79, 5.04, 4.46, 3.77, and 2.19 times, respectively. All of them have the characteristics of short sequence and strong activity. Among them, the murine homologous sequence of En4_622 (hTRPM1), mEn4_876 (mTRPM1) has similar expression activity (relative expression multiples: 4.73 times).

### 3.3 test results of combination of enhancer + core promoter

For the combined sequences: En4_284*2-P750(hTRPM1), En4_400-P750(hTRPM1), En4_160-P750(hTRPM1), En1_480-En4_160-P750(hTRPM1), En2_460-En4_160-P750(hTRPM1), and En4_622-P1061(hTRPM1), the test results show that the above promoters all have high promoter activity, and the expression intensity multiples relative to P750 are 6.77, 6.28, 4.98, 5.61, 6.96, and 8.54 times, respectively.

### Example 4. Intravitreal injection of rAAV2-NN to detect the expression specificity of the hTRPM1 promoter in mouse rod bipolar cells

To verify the expression specificity of the hTRPM1 series promoters in the retina and ON-bipolar cells, we injected the hTRPM1 series promoter-related AAV viruses described in Examples 2 and 3 into the vitreous cavity of mice. One month after the injection, the retinas were harvested and immunofluorescence staining was performed to detect the fluorescence expression intensity of the promoters in the retina.

As shown in Fig. 5, the promoter activities of the hTRPM1 series of novel promoters (such as P750 (hTRPM1), P1061 (hTRPM1), En4_622-P550 (hTRPM1), En4_400-P750 (hTRPM1), En2_460-En4_160-P750 (hTRPM1), En4_622-P1061 (hTRPM1)) and the prior art hGRM6 (1243) are mainly distributed in the inner nuclear layer (INL) and inner plexiform layer (IPL), indicating that they are mainly located in bipolar cells. The average fluorescence intensity (sum of fluorescence intensity/area) was analyzed using ImageJ, as shown in A of Fig. 6, wherein the expression activity of the exemplary P550 is almost invisible, while P750(hTRPM1) and P1061(hTRPM1) can be expressed in a small number of cells, with average GFP fluorescence intensities of 0.00AU (±0.01AU), 11.90AU (±1.85AU), and 12.33AU (±0.58AU), respectively. In addition, En4_622-P550 (hTRPM1), En4_400-P750 (hTRPM1), En2_460-En4_160-P750 (hTRPM1), En4_622-P1061 (hTRPM1) and hGRM6 (1243) are expressed at a high abundance in the retina, which are consistent with the results measured in the NanoLuc experiment. The GFP fluorescence intensities are 17.67AU (±1.53AU), 19.00AU (±2.00AU), 18.33AU (±3.215AU), 20.17AU (±2.26AU) and 19.13AU (±6.03AU), respectively.

The expression specificity of the promoter in ON-bipolar cells was detected by co-labeling GFP antibody and PKCα antibody. PKCα antibody can mark rod bipolar cells, which are a type of ON-bipolar cells. As shown in B of Fig. 6, GFP(+)PKCα(+)/PKCα(+) represents the expression ratio of the promoter-mediated GFP reporter gene in rod bipolar cells. The hTRPM1 series of novel promoters involved in the present application (such as P550 (hTRPM1), P750 (hTRPM1), P1061 (hTRPM1), En4_622-P550 (hTRPM1), En4_400-P750 (hTRPM1), En2_460-En4_160-P750 (hTRPM1), En4_622-P1061 (hTRPM1)) have expression activity in 0.003% (±0.01%), 36.00% (±9.839%), 43.49% (±18.03%), 57.33% (±6.81%), 60.00% (±10.00%), 62.33% (±9.29%), and 67.18% (±23.96%) of rod bipolar cells (labeled with PKCα antibody), respectively. Almost no activity is observed in the photoreceptor layer and ganglion cell layer. The hGRM6 (1243) staining results are consistent with those reported in the literature (PMID: 32258214), and the co-labeled cells account for 78.23% (± 9.69%) of the rod bipolar cells. The hTRPM1 series of novel promoters involved in this application (such as En4_622-P550 (hTRPM1), En4_400-P750 (hTRPM1), En2_460-En4_160-P750 (hTRPM1), En4_622-P1061 (hTRPM1) promoters) can efficiently initiate gene expression in ON bipolar cells and have potential application value in the treatment of retinal diseases.

### Example 5. Detection of expression activity of hTRPM1 series promoters in humanretinal cells

To verify the expression activity and specificity of the hTRPM1 promoter in human cells, we used AAV virus to infect human retinal organoids cultured and induced in vitro. Human retinal organoids are highly similar to human retina in terms of cell morphology, function, and developmental status, and can therefore be used to assist in determining the expression characteristics of the promoter in the human retina. As shown in Fig. 7, human retinal organoids differentiated for 200 days were mixed and co-cultured with the test virus of the hTRPM1 series promoter at a titer of 1E+11 vg/organoid. The expression of the promoter in human retinal organoids was detected by immunofluorescence staining 10 days after transduction. Human retinal organoid cells were labeled with GFP antibody, PKCα antibody (rod bipolar cells), and DAPI nuclear dye to determine the activity of the promoter in human ON-bipolar cells. GFP mediated by the hTRPM1 series promoter (such as the En400-P750 (hTRPM1) promoter) can be effectively expressed in ON-bipolar cells of human retinal organoids and has a high co-labeling with the rod bipolar cells of human retinal organoids. The hTRPM1 series promoter (such as the En400-P750 (hTRPM1) promoter) has similar activity to the prior art hGRM6 (1243) promoter in human retinal organoids, and both have the potential for application in human retinal cells and tissues.

### Example 6. Restoring vision of rd10 mice using hTRPM1 series promoters combined with photosensitive proteins

In order to verify the application of hTRPM1 series promoter in optogenetic gene therapy technology, it was fused with PsCatCh2.0 photosensitive protein (PMID: 35430811) gene and expression vector was constructed. The vector was packaged into AAV2-NN virus, and injected into 8-week-old wild-type rd10 mice through intravitreal injection. This example used the optokinetic response to evaluate the therapeutic effect of mice with retinitis pigmentosa. The results are shown in Fig. 8, the average visual acuity of rd10 mice treated with hTRPM1 series promoter-fused light-activated channel proteins (such as pAAV-En4_622-P550(hTRPM1)-PsCatCh2.0-WPRE-hGHpA, pAAV-En4_400-P750(hTRPM1)-PsCatCh2.0-WPRE-hGHpA and pAAV-En4_622-P1061(hTRPM1)-PsCatCh2.0-WPRE-hGHpA) is significantly improved compared with untreated rd10 mice. Specifically, the average maximum visual acuity of the two exemplary combinations are 0.18c/d (±0.06c/d), 0.19c/d (±0.07c/d), and 0.2c/d (±0.08c/d), respectively. The average maximum visual acuity of untreated rd10 mice is 0.07c/d (±0.07c/d, n=5), and the average maximum visual acuity of the WT group is 0.46c/d (±0.04c/d). Although the visual acuity of rd10 mice after treatment does not recover to the level of that of WT mice, it is still significantly improved compared with untreated rd10 mice, indicating that the hTRPM1 series promoters (such as En4_622-P550 (hTRPM1), En4_400-P750 (hTRPM1), En4_622-P1061 (hTRPM1)) have extremely high application value in the treatment of retinal diseases characterized by retinal photoreceptor degeneration or apoptosis (such as retinitis pigmentosa, Stargardt disease, retinal macular degeneration, etc.), or other diseases for which the therapeutic effects are achieved by expressing therapeutic genes in bipolar cells.

**Table 1: Sequence Listing**

| SEQ ID NO. | Name | Sequence (5'-3') |
|---|---|---|
| 1 | P1061 (hTRP M1) | TGTGTTGGTTGTAAGCCAGACCCCAAGAGGTGGAGGTCATGCCTCCACCCTCCAAACTTCCACAGCCGACAGGC CTCCCAGAGGAGGTGGTGGGTCCTGGCCTCACAGCTATGAGGGCTCCACAGCCTTCTATTATTTTATTTTCAGCTT GCTGGTCCTTAGATAAAAATCTATCTAAGACGATGGGAATACATGGCCTGTTCTCTCTGAGCTCCAGTCTGTTCCC TTGCATGTGGGAGGTGACATGCATGTGCCTGTAAGAGACAGAGGAGGGTCGGCAGCAAGGGTGGTACACTTCAG GCCAGGCTTGTGCTGCAAAGACCCATGACCCCATCTGCCTTTAAGAGGGGCAAGACAGGCACCCCTGAAATCAG AAATGACACAGAGAGCCCCACACACAGAGCAGCACAGAACAGACGGGCCACCGGCCACAGGATCTTCAGAAG AAGGCTTGCTGTGTCGCTGCCAGATTAACGGGGCGAAAATGCGCACTGGCAGCTCCAAGGAGAGAGCTGAGAC CCACAGCCTGGGCCTGGATGCAAATTCTTCTCTGAGACTCAGGCGGTTGGCCAGGTGGGCACCGGGCCTCTGGG ATCTGGATGCCACCCTCCAAGGGGCCTCAGATGCCAGCAGCAAGGGGCTGTCTGCCCTCGCAATGCGCCTTTCCA GACGCCCAAATCCTTCCCATTTGACCACCCTTTGCAGCCACCACTTTCTCAAAGTAGATTTACTTTCAATCTCCATT TGCTAAATCTCTTCCCTCTAAACCTTTTGGGGATGGAGGGATTAAGCCTGATTACAGATGTTGGGGGTGGTAGCAG GAGTCGGGCAGGAGGGGCCACCAAAAGCCAGCACATGCTCCTCCTAGGCTGAACCAGCCTCCGGCCGGCCTCG CCCCAGGATCTCGGCCCCCTCGCCCAGTGCGTGGGGCTGCCGCCGAGTGTGGGGCTGCCGCCGAGTGTGTGGCT CCGCTCCTGCCCACCCAGGGAGGGCAGCCCCTGTGCCACTGGCCTGGCCACCCGAGGGAGTCAGCAGGGTGGC TCACAGGCTCACTGAGAGAG |
| 2 | P750( hTRP M1) | GCTGCAAAGACCCATGACCCCATCTGCCTTTAAGAGGGGCAAGACAGGCACCCCTGAAATCAGAAATGACACAG AGAGCCCCACACACAGAGCAGCACAGAACAGACGGGCCACCGGCCACAGGATCTTCAGAAGAAGGCTTGCTGT GTCGCTGCCAGATTAACGGGGCGAAAATGCGCACTGGCAGCTCCAAGGAGAGAGCTGAGACCCACAGCCTGGG CCTGGATGCAAATTCTTCTCTGAGACTCAGGCGGTTGGCCAGGTGGGCACCGGGCCTCTGGGATCTGGATGCCAC CCTCCAAGGGGCCTCAGATGCCAGCAGCAAGGGGCTGTCTGCCCTCGCAATGCGCCTTTCCAGACGCCCAAATC CTTCCCATTTGACCACCCTTTGCAGCCACCACTTTCTCAAAGTAGATTTACTTTCAATCTCCATTTGCTAAATCTCT TCCCTCTAAACCTTTTGGGGATGGAGGGATTAAGCCTGATTACAGATGTTGGGGGTGGTAGCAGGAGTCGGGCAG GAGGGGCCACCAAAAGCCAGCACATGCTCCTCCTAGGCTGAACCAGCCTCCGGCCGGCCTCGCCCCAGGATCTC GGCCCCCTCGCCCAGTGCGTGGGGCTGCCGCCGAGTGTGGGGCTGCCGCCGAGTGTGTGGCTCCGCTCCTGCCC ACCCAGGGAGGGCAGCCCCTGTGCCACTGGCCTGGCCACCCGAGGGAGTCAGCAGGGTGGCTCACAGGCTCAC TGAGAGAG |
| 3 | P550( hTRP M1) | GCTGAGACCCACAGCCTGGGCCTGGATGCAAATTCTTCTCTGAGACTCAGGCGGTTGGCCAGGTGGGCACCGGG CCTCTGGGATCTGGATGCCACCCTCCAAGGGGCCTCAGATGCCAGCAGCAAGGGGCTGTCTGCCCTCGCAATGC GCCTTTCCAGACGCCCAAATCCTTCCCATTTGACCACCCTTTGCAGCCACCACTTTCTCAAAGTAGATTTACTTTC AATCTCCATTTGCTAAATCTCTTCCCTCTAAACCTTTTGGGGATGGAGGGATTAAGCCTGATTACAGATGTTGGGG GTGGTAGCAGGAGTCGGGCAGGAGGGGCCACCAAAAGCCAGCACATGCTCCTCCTAGGCTGAACCAGCCTCCG GCCGGCCTCGCCCCAGGATCTCGGCCCCCTCGCCCAGTGCGTGGGGCTGCCGCCGAGTGTGGGGCTGCCGCCGA GTGTGTGGCTCCGCTCCTGCCCACCCAGGGAGGGCAGCCCCTGTGCCACTGGCCTGGCCACCCGAGGGAGTCAG CAGGGTGGCTCACAGGCTCACTGAGAGAG |
| 4 | En1_8 02(hT RPM1 ) | TTTATAGCATAAATTGTCTATTCTCTGCAGTGTATTTTTTAGCCCTCCTCCCCCAGAAAATGTAATTGGAAATATCAA CTCATGTTGAATGTGTTTGCTGTACCTGTTTATGTTTTATTGATTATTTTCTGGGAGAAGTTTGGAGTTGTGTTTGGT TTGGCTCTGTGCAACCTCATTTATTTGCTCTGTAAAGCTGATAAGCACAAGAAGTGAAATGACACTCAGGCATTTT AAGATGCGAGTCATCTTCATTCTGAACTAAGTGAAAACAAAACAGATTTTCTATCAACCTAAAATCCTCCTACTGG GGGCAGTAGATGTTTTTAGAAAATTGGCTTTGGCTTTGATTGACAATGTTTGGAAGATGTCCTATAATTTGAGAGT GTATGTGTGTGTGTCTGGGTGTGTGTGTGTAAATGCTGGACCTGGGAAGATGGAAGCCCTCAGCCAGGAGAATAA GAGGACACAATACCCCCAAGCCTTCCATGACCTAGAGATTAAGGGGATTTAACGTCTTAAAACCTCCATTAAGTA GACAGTAGCAAGAGACATGTTGCCCTCAGTGATATGCAAAGGCATATCTGATGGGTCAGTATCTGCCATCCTCCTC TTTTGCAAGCTCTGAGGTTAGACTCAGCCATGGGTGAAGGAATTACAGCATCCATTGGGAGCAGGCAGAGTCCA GGAAAGAGATTTTTCTGTTACCTCAGATGCCCATCGCGCCAGGAGCAATTTTCCCAGCCTCTTGTAGAAAGCACAT ATTAGTATCTATCTCACTGCTTTTCAGAAACTGTATCTTCAA |
| 5 | En1_4 80(hT RPM1) | TCCCCCAGAAAATGTAATTGGAAATATCAACTCATGTTGAATGTGTTTGCTGTACCTGTTTATGTTTTATTGATTATT TTCTGGGAGAAGTTTGGAGTTGTGTTTGGTTTGGCTCTGTGCAACCTCATTTATTTGCTCTGTAAAGCTGATAAGC ACAAGAAGTGAAATGACACTCAGGCATTTTAAGATGCGAGTCATCTTCATTCTGAACTAAGTGAAAACAAAACA GATTTTCTATCAACCTAAAATCCTCCTACTGGGGGCAGTAGATGTTTTTAGAAAATTGGCTTTGGCTTTGATTGACA ATGTTTGGAAGATGTCCTATAATTTGAGAGTGTATGTGTGTGTGTCTGGGTGTGTGTGTGTAAATGCTGGACCTGG GAAGATGGAAGCCCTCAGCCAGGAGAATAAGAGGACACAATACCCCCAAGCCTTCCATGACCTAGAGATTAAGG GGATTTAACGTCTTAAAACCTCCAT |
| 6 | En2_1 251(h TRPM 1) | TGAAGGGGGGAGAGCAGGGATTAGAAGCACTCAGAAGGGGCTGAGAGTCATGTGGGGCTCACACTGCATTTGC AGCTGGGTTCACCCTGACCTCAGGCCCAACTTAGATGAGGAAGGATTAGCAGTAATTAGTGCCATGTGCCGCCTT CTCCCAGCTCCCCGGGGCACGAACACTGCCCAGCTGATGAGGGGATTCTGAAAGAACCATTATGTCCAATTGTCT CAATTATGCAAACCCTGCTGACATTTCCAGCCAGGGAAGGGCGGCTGGGTGGGAGGGGGCCATGGCGGGGCCAC TTCAAAGGAAAAGCTCTAGCTCCCCTACCTCTCTCACATCCTAAGGCTGCCTTTGTGGGATTCCACACAGAACAG CCTGGAAGCTTGGGGCCCTGGCTTCCTTTTCTGGCCTGGGAGTCAGGTCATGGGGCCATCGCTTCACAGCAATCA TGAGGGCCCAGGCCCAAGTGCTCACATGCTCCTCATGGGGACTGCTCCTCTTAAAGGGTGGGCCCTCCTCACCCA GCTCCCTGCCCTGGCCAAGGAGGAGGCTGAAAGAGCCTGAGCTGTGCCCTCTCCATTCCACTGCTGTGGCAGGT AAGCTCAGATGCGGGTAAGCACAGGTGAAGGGAGGTCAGGTGCAGGTATCCTCAGGTGTGGGTAAACTCAAGC GTGGACAAACTCAGGTGCAGATAACCACAGATGCAGTTAAGCTCAGGTGTTGGTAACCTCAGGTGCAGGTAGGC TCAGGTGCAAGTAAGAGCAGGTGCAGGCAAGCTCAGGTGTGCTTTCTGGGGCCTGTTACTCATCTCCTCTTGAGG CTGGCTGGACAGGACCCTATGGAGCAGCTGGTGAACCTGTGTTCAGCCTGGGTTCCAAGAGGCTGTCTCAGCAG AAGGGACCCACCAGGTTGATTCACAGAGGGGATAAGTACTCTGTCAGCTGGTCTCAAAGAGGCTGAAACCCTCT GAGCTTCCCTCAGGGCCAGCTTCTAAATCCAGGTGCCAGGGATCTGCTCAGGAGCCCCGGCTCCAGGCCTCAAC CTCTTGCCTGTCATTCCTTAGCATCTTACTTTGCTTCAGGGAAAAGGTGCACCTTCCTTAGAACCCCAGAAAATGC TGAAGTTCTAGGAGAGACAGGGTTTATACTTGTAAGCCTTTATCAGCTGATATTTCACCTGTGAAAGCCTTGGGGA AGAGATTGGTTACACGGAACAGGATCAGGAGGGGAGATTACTGCTCCCTCTCACTGCTG |
| 7 | En2_8 80(hT RPM1 ) | TGTGGGGCTCACACTGCATTTGCAGCTGGGTTCACCCTGACCTCAGGCCCAACTTAGATGAGGAAGGATTAGCAG TAATTAGTGCCATGTGCCGCCTTCTCCCAGCTCCCCGGGGCACGAACACTGCCCAGCTGATGAGGGGATTCTGAA AGAACCATTATGTCCAATTGTCTCAATTATGCAAACCCTGCTGACATTTCCAGCCAGGGAAGGGCGAGAGCCTGA GCTGTGCCCTCTCCATTCCACTGCTGTGGCAGGTAAGCTCAGATGCGGGTAAGCACAGGTGAAGGGAGGTCAGG TGCAGGTATCCTCAGGTGTGGGTAAACTCAAGCGTGGACAAACTCAGGTGCAGATAACCACAGATGCAGTTAAG CTCAGGTGTTGGTAACCTCAGGTGCAGGTAGGCTCAGGTGCAAGTAAGAGCAGGTGCAGGCAAGCTCAGGTGTG CTTTCTGGGGCCTGTTACTCATCTCCTCTTGAGGCTGGCTGGACAGGACCCTATGGAGCAGCTGGTGAACCTGTG TTCAGCCTGGGTTCCAAGAGGCTGTCTCAGCAGAAGGGACCCACCAGGTTGATTCACAGAGGGGATAAGTACTC TGTCAGCTGGTCTCAAAGAGGCTGAAACCCTCTGAGCTTCCCTCAGGGCCAGCTTCTAAATCCAGGTGCCAGGG ATCTGCTCAGGAGCCCCGGCTCCAGGCCTCAACCTCTTGCCTGTCATTCCTTAGCATCTTACTTTGCTTCAGGGAA AAGGTGCACCTTCCTTAGAACCCCAGAAAATGCTGAAGTTCTAGGAGAGACAGGGTTTATACTTGTAAGCCTTTA TCAGCTGATATTTCACCTGTGAAAGCCTTGGGGAAGAGATTGGTTACACGGAACAGGATC |
| 8 | En2_4 60(hT RPM1 ) | TGTGGGGCTCACACTGCATTTGCAGCTGGGTTCACCCTGACCTCAGGCCCAACTTAGATGAGGAAGGATTAGCAG TAATTAGTGCCATGTGCCGCCTTCTCCCAGCTCCCCGGGGCACGAACACTGCCCAGCTGATGAGGGGATTCTGAA AGAACCATTATGGGGATAAGTACTCTGTCAGCTGGTCTCAAAGAGGCTGAAACCCTCTGAGCTTCCCTCAGGGCC AGCTTCTAAATCCAGGTGCCAGGGATCTGCTCAGGAGCCCCGGCTCCAGGCCTCAACCTCTTGCCTGTCATTCCT TAGCATCTTACTTTGCTTCAGGGAAAAGGTGCACCTTCCTTAGAACCCCAGAAAATGCTGAAGTTCTAGGAGAGA CAGGGTTTATACTTGTAAGCCTTTATCAGCTGATATTTCACCTGTGAAAGCCTTGGGGAAGAGATTGGTTACACGG AACAGGATC |
| 9 | En3_2 77(hT RPM1 ) | CAGAAAGTTGACAAACTACAGCTGAGTATCAGGTCGGCACTGGAGGCGGCCAGGTGGGGGCAGCAGAGAGCTG AGTGGGCAGAAACCGGGGCTCATAGGCGGTGAGGTTCTGTCAACGGAGCTGCAAGAGAAGTAACCCTGCAGGG TCCCACCAAATGACCCAAAGTGGAAAGGGCCAGGCGGCCCGGGTCCCTTACTGGACAGATCTGCGCAGTAAGCA CAAGTCAGCACTGTTGCTGTGGCACTGGCACCATCAGTACTTGACCTAGGATCACCT |
| 10 | En4_6 22(hT RPM1 ) | GAGCACTTGAGCTTTCAGGGTGCAGGGCCTGTGGGAAACGGGCCATGGACCTCCTCGGGGGGATGGAGGCCCCT CAGTCGGGAGGTGCAAGAAAGAGGAGCTGCTTTTGGGGTCACAGAGGGGCCAGGGGAGGCAGAGCTTGTGCCC AACCTTGGGGTCAACCCGGCTGCCAATAGGCCCATGCAAACCCAAGACAGTGGGCCATCAGGAAGGCACTGTGG AGGGGTAATCCGCGTAATGGCCCAGAACACGGCGTAACAGCTGGCTGTGGCTAATGCTCCTCAATATCTGCTTTTA TGTGATTAATGGCTGTGGATAAAACCTTGATCCCAGGAAAGCAGGCCCAGCGGCTAAGCCTAGCCAAGAGCACC ACTTTGGTTATGCTGGGAGCCCGAGTCATTTTGGCGATTTTCATTTTAACCAAGCTGTCATTTTAGTATGTGAGGAA AAAAAATAAAAGTGGTAGGAACTACACTCACAGAGAGCCCAGAGAGGGGGCAGCAGGGGGGTGGGGGCGAGC CTGGTGCTGGAAGTGTGAAAACAGCTGGGGGCCCAAGTGCCCTGACTCCCCTGGCCACCCACTCACTGCTTCCA CCCACTCTCACTTCCCTTGGGTTCACAC |
| 11 | En4_4 00(hT RPM1 ) | GCAAGAAAGAGGAGCTGCTTTTGGGGTCACAGAGGGGCCAGGGGAGGCAGAGCTTGTGCCCAACCTTGGGGTC AACCCGGCTGCCAATAGGCCCATGCAAACCCAAGACAGTGGGCCATCAGGAAGGCACTGTGGAGGGGTAATCCG CGTAATGGCCCAGAACACGGCGTAACAGCTGGCTGTGGCTAATGCTCCTCAATATCTGCTTTTATGTGATTAATGG CTGTGGATAAAACCTTGATCCCAGGAAAGCAGGCCCAGCGGCTAAGCCTAGCCAAGAGCACCACTTTGGTTATG CTGGGAGCCCGAGTCATTTTGGCGATTTTCATTTTAACCAAGCTGTCATTTTAGTATGTGAGGAAAAAAAATAAAA GTGGTAGGAACTACACTCACAGAGAGC |
| 12 | En4_2 84(hT RPM1 ) | CATCAGGAAGGCACTGTGGAGGGGTAATCCGCGTAATGGCCCAGAACACGGCGTAACAGCTGGCTGTGGCTAAT GCTCCTCAATATCTGCTTTTATGTGATTAATGGCTGTGGATAAAACCTTGATCCCAGGAAAGCAGGCCCAGCGGCT AAGCCTAGCCAAGAGCACCACTTTGGTTATGCTGGGAGCCCGAGTCATTTTGGCGATTTTCATTTTAACCAAGCTG TCATTTTAGTATGTGAGGAAAAAAAATAAAAGTGGTAGGAACTACACTCACAGAGAGC |
| 13 | En4_1 60(hT RPM1 ) | CGGGGGGATGGAGGCCCCTCAGTCGGGAGGTGCAAGAAAGAGGAGCTGCTTTTGGGGTCACAGAGGGGCCAGG GGAGGCAGAGCTTGTGCCCAACCTTGGGGTCAGCTGGCTGTGGCTAATGCTCCTCAATATCTGCTTTTATGTGATT AATGGCTGTGG |
| 14 | En4 5 6(hTR PM1) | AGCTGGCTGTGGCTAATGCTCCTCAATATCTGCTTTTATGTGATTAATGGCTGTGG |
| 15 | En1_8 02-P5 50(hT RPM1 ) | TTTATAGCATAAATTGTCTATTCTCTGCAGTGTATTTTTTAGCCCTCCTCCCCCAGAAAATGTAATTGGAAATATCAA CTCATGTTGAATGTGTTTGCTGTACCTGTTTATGTTTTATTGATTATTTTCTGGGAGAAGTTTGGAGTTGTGTTTGGT TTGGCTCTGTGCAACCTCATTTATTTGCTCTGTAAAGCTGATAAGCACAAGAAGTGAAATGACACTCAGGCATTTT AAGATGCGAGTCATCTTCATTCTGAACTAAGTGAAAACAAAACAGATTTTCTATCAACCTAAAATCCTCCTACTGG GGGCAGTAGATGTTTTTAGAAAATTGGCTTTGGCTTTGATTGACAATGTTTGGAAGATGTCCTATAATTTGAGAGT GTATGTGTGTGTGTCTGGGTGTGTGTGTGTAAATGCTGGACCTGGGAAGATGGAAGCCCTCAGCCAGGAGAATAA GAGGACACAATACCCCCAAGCCTTCCATGACCTAGAGATTAAGGGGATTTAACGTCTTAAAACCTCCATTAAGTA GACAGTAGCAAGAGACATGTTGCCCTCAGTGATATGCAAAGGCATATCTGATGGGTCAGTATCTGCCATCCTCCTC TTTTGCAAGCTCTGAGGTTAGACTCAGCCATGGGTGAAGGAATTACAGCATCCATTGGGAGCAGGCAGAGTCCA GGAAAGAGATTTTTCTGTTACCTCAGATGCCCATCGCGCCAGGAGCAATTTTCCCAGCCTCTTGTAGAAAGCACAT ATTAGTATCTATCTCACTGCTTTTCAGAAACTGTATCTTCAAGCTGAGACCCACAGCCTGGGCCTGGATGCAAATT CTTCTCTGAGACTCAGGCGGTTGGCCAGGTGGGCACCGGGCCTCTGGGATCTGGATGCCACCCTCCAAGGGGCC TCAGATGCCAGCAGCAAGGGGCTGTCTGCCCTCGCAATGCGCCTTTCCAGACGCCCAAATCCTTCCCATTTGACC ACCCTTTGCAGCCACCACTTTCTCAAAGTAGATTTACTTTCAATCTCCATTTGCTAAATCTCTTCCCTCTAAACCTT TTGGGGATGGAGGGATTAAGCCTGATTACAGATGTTGGGGGTGGTAGCAGGAGTCGGGCAGGAGGGGCCACCAA AAGCCAGCACATGCTCCTCCTAGGCTGAACCAGCCTCCGGCCGGCCTCGCCCCAGGATCTCGGCCCCCTCGCCCA GTGCGTGGGGCTGCCGCCGAGTGTGGGGCTGCCGCCGAGTGTGTGGCTCCGCTCCTGCCCACCCAGGGAGGGC AGCCCCTGTGCCACTGGCCTGGCCACCCGAGGGAGTCAGCAGGGTGGCTCACAGGCTCACTGAGAGAG |
| 16 | En1_4 80-P5 50(hT RPM1 ) | TCCCCCAGAAAATGTAATTGGAAATATCAACTCATGTTGAATGTGTTTGCTGTACCTGTTTATGTTTTATTGATTATT TTCTGGGAGAAGTTTGGAGTTGTGTTTGGTTTGGCTCTGTGCAACCTCATTTATTTGCTCTGTAAAGCTGATAAGC ACAAGAAGTGAAATGACACTCAGGCATTTTAAGATGCGAGTCATCTTCATTCTGAACTAAGTGAAAACAAAACA GATTTTCTATCAACCTAAAATCCTCCTACTGGGGGCAGTAGATGTTTTTAGAAAATTGGCTTTGGCTTTGATTGACA ATGTTTGGAAGATGTCCTATAATTTGAGAGTGTATGTGTGTGTGTCTGGGTGTGTGTGTGTAAATGCTGGACCTGG GAAGATGGAAGCCCTCAGCCAGGAGAATAAGAGGACACAATACCCCCAAGCCTTCCATGACCTAGAGATTAAGG GGATTTAACGTCTTAAAACCTCCATGCTGAGACCCACAGCCTGGGCCTGGATGCAAATTCTTCTCTGAGACTCAG GCGGTTGGCCAGGTGGGCACCGGGCCTCTGGGATCTGGATGCCACCCTCCAAGGGGCCTCAGATGCCAGCAGCA AGGGGCTGTCTGCCCTCGCAATGCGCCTTTCCAGACGCCCAAATCCTTCCCATTTGACCACCCTTTGCAGCCACC ACTTTCTCAAAGTAGATTTACTTTCAATCTCCATTTGCTAAATCTCTTCCCTCTAAACCTTTTGGGGATGGAGGGAT TAAGCCTGATTACAGATGTTGGGGGTGGTAGCAGGAGTCGGGCAGGAGGGGCCACCAAAAGCCAGCACATGCTC CTCCTAGGCTGAACCAGCCTCCGGCCGGCCTCGCCCCAGGATCTCGGCCCCCTCGCCCAGTGCGTGGGGCTGCC GCCGAGTGTGGGGCTGCCGCCGAGTGTGTGGCTCCGCTCCTGCCCACCCAGGGAGGGCAGCCCCTGTGCCACTG GCCTGGCCACCCGAGGGAGTCAGCAGGGTGGCTCACAGGCTCACTGAGAGAG |
| 17 | En2_1 251-P 550(h TRPM 1) | TGAAGGGGGGAGAGCAGGGATTAGAAGCACTCAGAAGGGGCTGAGAGTCATGTGGGGCTCACACTGCATTTGC AGCTGGGTTCACCCTGACCTCAGGCCCAACTTAGATGAGGAAGGATTAGCAGTAATTAGTGCCATGTGCCGCCTT CTCCCAGCTCCCCGGGGCACGAACACTGCCCAGCTGATGAGGGGATTCTGAAAGAACCATTATGTCCAATTGTCT CAATTATGCAAACCCTGCTGACATTTCCAGCCAGGGAAGGGCGGCTGGGTGGGAGGGGGCCATGGCGGGGCCAC TTCAAAGGAAAAGCTCTAGCTCCCCTACCTCTCTCACATCCTAAGGCTGCCTTTGTGGGATTCCACACAGAACAG CCTGGAAGCTTGGGGCCCTGGCTTCCTTTTCTGGCCTGGGAGTCAGGTCATGGGGCCATCGCTTCACAGCAATCA TGAGGGCCCAGGCCCAAGTGCTCACATGCTCCTCATGGGGACTGCTCCTCTTAAAGGGTGGGCCCTCCTCACCCA GCTCCCTGCCCTGGCCAAGGAGGAGGCTGAAAGAGCCTGAGCTGTGCCCTCTCCATTCCACTGCTGTGGCAGGT AAGCTCAGATGCGGGTAAGCACAGGTGAAGGGAGGTCAGGTGCAGGTATCCTCAGGTGTGGGTAAACTCAAGC GTGGACAAACTCAGGTGCAGATAACCACAGATGCAGTTAAGCTCAGGTGTTGGTAACCTCAGGTGCAGGTAGGC TCAGGTGCAAGTAAGAGCAGGTGCAGGCAAGCTCAGGTGTGCTTTCTGGGGCCTGTTACTCATCTCCTCTTGAGG CTGGCTGGACAGGACCCTATGGAGCAGCTGGTGAACCTGTGTTCAGCCTGGGTTCCAAGAGGCTGTCTCAGCAG AAGGGACCCACCAGGTTGATTCACAGAGGGGATAAGTACTCTGTCAGCTGGTCTCAAAGAGGCTGAAACCCTCT GAGCTTCCCTCAGGGCCAGCTTCTAAATCCAGGTGCCAGGGATCTGCTCAGGAGCCCCGGCTCCAGGCCTCAAC CTCTTGCCTGTCATTCCTTAGCATCTTACTTTGCTTCAGGGAAAAGGTGCACCTTCCTTAGAACCCCAGAAAATGC TGAAGTTCTAGGAGAGACAGGGTTTATACTTGTAAGCCTTTATCAGCTGATATTTCACCTGTGAAAGCCTTGGGGA AGAGATTGGTTACACGGAACAGGATCAGGAGGGGAGATTACTGCTCCCTCTCACTGCTGGCTGAGACCCACAGC CTGGGCCTGGATGCAAATTCTTCTCTGAGACTCAGGCGGTTGGCCAGGTGGGCACCGGGCCTCTGGGATCTGGAT GCCACCCTCCAAGGGGCCTCAGATGCCAGCAGCAAGGGGCTGTCTGCCCTCGCAATGCGCCTTTCCAGACGCCC AAATCCTTCCCATTTGACCACCCTTTGCAGCCACCACTTTCTCAAAGTAGATTTACTTTCAATCTCCATTTGCTAAA TCTCTTCCCTCTAAACCTTTTGGGGATGGAGGGATTAAGCCTGATTACAGATGTTGGGGGTGGTAGCAGGAGTCG GGCAGGAGGGGCCACCAAAAGCCAGCACATGCTCCTCCTAGGCTGAACCAGCCTCCGGCCGGCCTCGCCCCAG GATCTCGGCCCCCTCGCCCAGTGCGTGGGGCTGCCGCCGAGTGTGGGGCTGCCGCCGAGTGTGTGGCTCCGCTC CTGCCCACCCAGGGAGGGCAGCCCCTGTGCCACTGGCCTGGCCACCCGAGGGAGTCAGCAGGGTGGCTCACAG GCTCACTGAGAGAG |
| 18 | En2_8 80-P5 50(hT RPM1 ) | TGTGGGGCTCACACTGCATTTGCAGCTGGGTTCACCCTGACCTCAGGCCCAACTTAGATGAGGAAGGATTAGCAG TAATTAGTGCCATGTGCCGCCTTCTCCCAGCTCCCCGGGGCACGAACACTGCCCAGCTGATGAGGGGATTCTGAA AGAACCATTATGTCCAATTGTCTCAATTATGCAAACCCTGCTGACATTTCCAGCCAGGGAAGGGCGAGAGCCTGA GCTGTGCCCTCTCCATTCCACTGCTGTGGCAGGTAAGCTCAGATGCGGGTAAGCACAGGTGAAGGGAGGTCAGG TGCAGGTATCCTCAGGTGTGGGTAAACTCAAGCGTGGACAAACTCAGGTGCAGATAACCACAGATGCAGTTAAG CTCAGGTGTTGGTAACCTCAGGTGCAGGTAGGCTCAGGTGCAAGTAAGAGCAGGTGCAGGCAAGCTCAGGTGTG CTTTCTGGGGCCTGTTACTCATCTCCTCTTGAGGCTGGCTGGACAGGACCCTATGGAGCAGCTGGTGAACCTGTG TTCAGCCTGGGTTCCAAGAGGCTGTCTCAGCAGAAGGGACCCACCAGGTTGATTCACAGAGGGGATAAGTACTC TGTCAGCTGGTCTCAAAGAGGCTGAAACCCTCTGAGCTTCCCTCAGGGCCAGCTTCTAAATCCAGGTGCCAGGG ATCTGCTCAGGAGCCCCGGCTCCAGGCCTCAACCTCTTGCCTGTCATTCCTTAGCATCTTACTTTGCTTCAGGGAA AAGGTGCACCTTCCTTAGAACCCCAGAAAATGCTGAAGTTCTAGGAGAGACAGGGTTTATACTTGTAAGCCTTTA TCAGCTGATATTTCACCTGTGAAAGCCTTGGGGAAGAGATTGGTTACACGGAACAGGATCGCTGAGACCCACAG CCTGGGCCTGGATGCAAATTCTTCTCTGAGACTCAGGCGGTTGGCCAGGTGGGCACCGGGCCTCTGGGATCTGGA TGCCACCCTCCAAGGGGCCTCAGATGCCAGCAGCAAGGGGCTGTCTGCCCTCGCAATGCGCCTTTCCAGACGCC CAAATCCTTCCCATTTGACCACCCTTTGCAGCCACCACTTTCTCAAAGTAGATTTACTTTCAATCTCCATTTGCTAA ATCTCTTCCCTCTAAACCTTTTGGGGATGGAGGGATTAAGCCTGATTACAGATGTTGGGGGTGGTAGCAGGAGTC GGGCAGGAGGGGCCACCAAAAGCCAGCACATGCTCCTCCTAGGCTGAACCAGCCTCCGGCCGGCCTCGCCCCA GGATCTCGGCCCCCTCGCCCAGTGCGTGGGGCTGCCGCCGAGTGTGGGGCTGCCGCCGAGTGTGTGGCTCCGCT CCTGCCCACCCAGGGAGGGCAGCCCCTGTGCCACTGGCCTGGCCACCCGAGGGAGTCAGCAGGGTGGCTCACA GGCTCACTGAGAGAG |
| 19 | En2_4 60-P5 50(hT RPM1 ) | TGTGGGGCTCACACTGCATTTGCAGCTGGGTTCACCCTGACCTCAGGCCCAACTTAGATGAGGAAGGATTAGCAG TAATTAGTGCCATGTGCCGCCTTCTCCCAGCTCCCCGGGGCACGAACACTGCCCAGCTGATGAGGGGATTCTGAA AGAACCATTATGGGGATAAGTACTCTGTCAGCTGGTCTCAAAGAGGCTGAAACCCTCTGAGCTTCCCTCAGGGCC AGCTTCTAAATCCAGGTGCCAGGGATCTGCTCAGGAGCCCCGGCTCCAGGCCTCAACCTCTTGCCTGTCATTCCT TAGCATCTTACTTTGCTTCAGGGAAAAGGTGCACCTTCCTTAGAACCCCAGAAAATGCTGAAGTTCTAGGAGAGA CAGGGTTTATACTTGTAAGCCTTTATCAGCTGATATTTCACCTGTGAAAGCCTTGGGGAAGAGATTGGTTACACGG AACAGGATCGCTGAGACCCACAGCCTGGGCCTGGATGCAAATTCTTCTCTGAGACTCAGGCGGTTGGCCAGGTG GGCACCGGGCCTCTGGGATCTGGATGCCACCCTCCAAGGGGCCTCAGATGCCAGCAGCAAGGGGCTGTCTGCCC TCGCAATGCGCCTTTCCAGACGCCCAAATCCTTCCCATTTGACCACCCTTTGCAGCCACCACTTTCTCAAAGTAGA TTTACTTTCAATCTCCATTTGCTAAATCTCTTCCCTCTAAACCTTTTGGGGATGGAGGGATTAAGCCTGATTACAGA TGTTGGGGGTGGTAGCAGGAGTCGGGCAGGAGGGGCCACCAAAAGCCAGCACATGCTCCTCCTAGGCTGAACC AGCCTCCGGCCGGCCTCGCCCCAGGATCTCGGCCCCCTCGCCCAGTGCGTGGGGCTGCCGCCGAGTGTGGGGCT GCCGCCGAGTGTGTGGCTCCGCTCCTGCCCACCCAGGGAGGGCAGCCCCTGTGCCACTGGCCTGGCCACCCGAG GGAGTCAGCAGGGTGGCTCACAGGCTCACTGAGAGAG |
| 20 | En3_2 77-P5 50(hT RPM1 ) | CAGAAAGTTGACAAACTACAGCTGAGTATCAGGTCGGCACTGGAGGCGGCCAGGTGGGGGCAGCAGAGAGCTG AGTGGGCAGAAACCGGGGCTCATAGGCGGTGAGGTTCTGTCAACGGAGCTGCAAGAGAAGTAACCCTGCAGGG TCCCACCAAATGACCCAAAGTGGAAAGGGCCAGGCGGCCCGGGTCCCTTACTGGACAGATCTGCGCAGTAAGCA CAAGTCAGCACTGTTGCTGTGGCACTGGCACCATCAGTACTTGACCTAGGATCACCTGCTGAGACCCACAGCCTG GGCCTGGATGCAAATTCTTCTCTGAGACTCAGGCGGTTGGCCAGGTGGGCACCGGGCCTCTGGGATCTGGATGCC ACCCTCCAAGGGGCCTCAGATGCCAGCAGCAAGGGGCTGTCTGCCCTCGCAATGCGCCTTTCCAGACGCCCAAA TCCTTCCCATTTGACCACCCTTTGCAGCCACCACTTTCTCAAAGTAGATTTACTTTCAATCTCCATTTGCTAAATCT CTTCCCTCTAAACCTTTTGGGGATGGAGGGATTAAGCCTGATTACAGATGTTGGGGGTGGTAGCAGGAGTCGGGC AGGAGGGGCCACCAAAAGCCAGCACATGCTCCTCCTAGGCTGAACCAGCCTCCGGCCGGCCTCGCCCCAGGATC TCGGCCCCCTCGCCCAGTGCGTGGGGCTGCCGCCGAGTGTGGGGCTGCCGCCGAGTGTGTGGCTCCGCTCCTGC CCACCCAGGGAGGGCAGCCCCTGTGCCACTGGCCTGGCCACCCGAGGGAGTCAGCAGGGTGGCTCACAGGCTC ACTGAGAGAG |
| 21 | En4_6 22-P5 50(hTRPM1 ) | GAGCACTTGAGCTTTCAGGGTGCAGGGCCTGTGGGAAACGGGCCATGGACCTCCTCGGGGGGATGGAGGCCCCT CAGTCGGGAGGTGCAAGAAAGAGGAGCTGCTTTTGGGGTCACAGAGGGGCCAGGGGAGGCAGAGCTTGTGCCC AACCTTGGGGTCAACCCGGCTGCCAATAGGCCCATGCAAACCCAAGACAGTGGGCCATCAGGAAGGCACTGTGG AGGGGTAATCCGCGTAATGGCCCAGAACACGGCGTAACAGCTGGCTGTGGCTAATGCTCCTCAATATCTGCTTTTA TGTGATTAATGGCTGTGGATAAAACCTTGATCCCAGGAAAGCAGGCCCAGCGGCTAAGCCTAGCCAAGAGCACC ACTTTGGTTATGCTGGGAGCCCGAGTCATTTTGGCGATTTTCATTTTAACCAAGCTGTCATTTTAGTATGTGAGGAA AAAAAATAAAAGTGGTAGGAACTACACTCACAGAGAGCCCAGAGAGGGGGCAGCAGGGGGGTGGGGGCGAGC CTGGTGCTGGAAGTGTGAAAACAGCTGGGGGCCCAAGTGCCCTGACTCCCCTGGCCACCCACTCACTGCTTCCA CCCACTCTCACTTCCCTTGGGTTCACACGCTGAGACCCACAGCCTGGGCCTGGATGCAAATTCTTCTCTGAGACT CAGGCGGTTGGCCAGGTGGGCACCGGGCCTCTGGGATCTGGATGCCACCCTCCAAGGGGCCTCAGATGCCAGCA GCAAGGGGCTGTCTGCCCTCGCAATGCGCCTTTCCAGACGCCCAAATCCTTCCCATTTGACCACCCTTTGCAGCC ACCACTTTCTCAAAGTAGATTTACTTTCAATCTCCATTTGCTAAATCTCTTCCCTCTAAACCTTTTGGGGATGGAGG GATTAAGCCTGATTACAGATGTTGGGGGTGGTAGCAGGAGTCGGGCAGGAGGGGCCACCAAAAGCCAGCACATG CTCCTCCTAGGCTGAACCAGCCTCCGGCCGGCCTCGCCCCAGGATCTCGGCCCCCTCGCCCAGTGCGTGGGGCTG CCGCCGAGTGTGGGGCTGCCGCCGAGTGTGTGGCTCCGCTCCTGCCCACCCAGGGAGGGCAGCCCCTGTGCCAC TGGCCTGGCCACCCGAGGGAGTCAGCAGGGTGGCTCACAGGCTCACTGAGAGAG |
| 22 | En4_4 00-P5 50(hT RPM1 ) | GCAAGAAAGAGGAGCTGCTTTTGGGGTCACAGAGGGGCCAGGGGAGGCAGAGCTTGTGCCCAACCTTGGGGTC AACCCGGCTGCCAATAGGCCCATGCAAACCCAAGACAGTGGGCCATCAGGAAGGCACTGTGGAGGGGTAATCCG CGTAATGGCCCAGAACACGGCGTAACAGCTGGCTGTGGCTAATGCTCCTCAATATCTGCTTTTATGTGATTAATGG CTGTGGATAAAACCTTGATCCCAGGAAAGCAGGCCCAGCGGCTAAGCCTAGCCAAGAGCACCACTTTGGTTATG CTGGGAGCCCGAGTCATTTTGGCGATTTTCATTTTAACCAAGCTGTCATTTTAGTATGTGAGGAAAAAAAATAAAA GTGGTAGGAACTACACTCACAGAGAGCGCTGAGACCCACAGCCTGGGCCTGGATGCAAATTCTTCTCTGAGACT CAGGCGGTTGGCCAGGTGGGCACCGGGCCTCTGGGATCTGGATGCCACCCTCCAAGGGGCCTCAGATGCCAGCA GCAAGGGGCTGTCTGCCCTCGCAATGCGCCTTTCCAGACGCCCAAATCCTTCCCATTTGACCACCCTTTGCAGCC ACCACTTTCTCAAAGTAGATTTACTTTCAATCTCCATTTGCTAAATCTCTTCCCTCTAAACCTTTTGGGGATGGAGG GATTAAGCCTGATTACAGATGTTGGGGGTGGTAGCAGGAGTCGGGCAGGAGGGGCCACCAAAAGCCAGCACATG CTCCTCCTAGGCTGAACCAGCCTCCGGCCGGCCTCGCCCCAGGATCTCGGCCCCCTCGCCCAGTGCGTGGGGCTG CCGCCGAGTGTGGGGCTGCCGCCGAGTGTGTGGCTCCGCTCCTGCCCACCCAGGGAGGGCAGCCCCTGTGCCAC TGGCCTGGCCACCCGAGGGAGTCAGCAGGGTGGCTCACAGGCTCACTGAGAGAG |
| 23 | En4_2 84-P5 50(hT RPM1 ) | CATCAGGAAGGCACTGTGGAGGGGTAATCCGCGTAATGGCCCAGAACACGGCGTAACAGCTGGCTGTGGCTAAT GCTCCTCAATATCTGCTTTTATGTGATTAATGGCTGTGGATAAAACCTTGATCCCAGGAAAGCAGGCCCAGCGGCT AAGCCTAGCCAAGAGCACCACTTTGGTTATGCTGGGAGCCCGAGTCATTTTGGCGATTTTCATTTTAACCAAGCTG TCATTTTAGTATGTGAGGAAAAAAAATAAAAGTGGTAGGAACTACACTCACAGAGAGCGCTGAGACCCACAGCC TGGGCCTGGATGCAAATTCTTCTCTGAGACTCAGGCGGTTGGCCAGGTGGGCACCGGGCCTCTGGGATCTGGATG CCACCCTCCAAGGGGCCTCAGATGCCAGCAGCAAGGGGCTGTCTGCCCTCGCAATGCGCCTTTCCAGACGCCCA AATCCTTCCCATTTGACCACCCTTTGCAGCCACCACTTTCTCAAAGTAGATTTACTTTCAATCTCCATTTGCTAAAT CTCTTCCCTCTAAACCTTTTGGGGATGGAGGGATTAAGCCTGATTACAGATGTTGGGGGTGGTAGCAGGAGTCGG GCAGGAGGGGCCACCAAAAGCCAGCACATGCTCCTCCTAGGCTGAACCAGCCTCCGGCCGGCCTCGCCCCAGGA TCTCGGCCCCCTCGCCCAGTGCGTGGGGCTGCCGCCGAGTGTGGGGCTGCCGCCGAGTGTGTGGCTCCGCTCCT GCCCACCCAGGGAGGGCAGCCCCTGTGCCACTGGCCTGGCCACCCGAGGGAGTCAGCAGGGTGGCTCACAGGC TCACTGAGAGAG |
| 24 | En4_1 60-P5 50(hT RPM1 ) | CGGGGGGATGGAGGCCCCTCAGTCGGGAGGTGCAAGAAAGAGGAGCTGCTTTTGGGGTCACAGAGGGGCCAGG GGAGGCAGAGCTTGTGCCCAACCTTGGGGTCAGCTGGCTGTGGCTAATGCTCCTCAATATCTGCTTTTATGTGATT AATGGCTGTGGGCTGAGACCCACAGCCTGGGCCTGGATGCAAATTCTTCTCTGAGACTCAGGCGGTTGGCCAGG TGGGCACCGGGCCTCTGGGATCTGGATGCCACCCTCCAAGGGGCCTCAGATGCCAGCAGCAAGGGGCTGTCTGC CCTCGCAATGCGCCTTTCCAGACGCCCAAATCCTTCCCATTTGACCACCCTTTGCAGCCACCACTTTCTCAAAGTA GATTTACTTTCAATCTCCATTTGCTAAATCTCTTCCCTCTAAACCTTTTGGGGATGGAGGGATTAAGCCTGATTACA GATGTTGGGGGTGGTAGCAGGAGTCGGGCAGGAGGGGCCACCAAAAGCCAGCACATGCTCCTCCTAGGCTGAA CCAGCCTCCGGCCGGCCTCGCCCCAGGATCTCGGCCCCCTCGCCCAGTGCGTGGGGCTGCCGCCGAGTGTGGGG CTGCCGCCGAGTGTGTGGCTCCGCTCCTGCCCACCCAGGGAGGGCAGCCCCTGTGCCACTGGCCTGGCCACCCG AGGGAGTCAGCAGGGTGGCTCACAGGCTCACTGAGAGAG |
| 25 | En4_5 6-P55 0(hTR PM1) | AGCTGGCTGTGGCTAATGCTCCTCAATATCTGCTTTTATGTGATTAATGGCTGTGGGCTGAGACCCACAGCCTGGG CCTGGATGCAAATTCTTCTCTGAGACTCAGGCGGTTGGCCAGGTGGGCACCGGGCCTCTGGGATCTGGATGCCAC CCTCCAAGGGGCCTCAGATGCCAGCAGCAAGGGGCTGTCTGCCCTCGCAATGCGCCTTTCCAGACGCCCAAATC CTTCCCATTTGACCACCCTTTGCAGCCACCACTTTCTCAAAGTAGATTTACTTTCAATCTCCATTTGCTAAATCTCT TCCCTCTAAACCTTTTGGGGATGGAGGGATTAAGCCTGATTACAGATGTTGGGGGTGGTAGCAGGAGTCGGGCAG GAGGGGCCACCAAAAGCCAGCACATGCTCCTCCTAGGCTGAACCAGCCTCCGGCCGGCCTCGCCCCAGGATCTC GGCCCCCTCGCCCAGTGCGTGGGGCTGCCGCCGAGTGTGGGGCTGCCGCCGAGTGTGTGGCTCCGCTCCTGCCC ACCCAGGGAGGGCAGCCCCTGTGCCACTGGCCTGGCCACCCGAGGGAGTCAGCAGGGTGGCTCACAGGCTCAC TGAGAGAG |
| 26 | En4_2 84*2-P750( hTRP M1) | CATCAGGAAGGCACTGTGGAGGGGTAATCCGCGTAATGGCCCAGAACACGGCGTAACAGCTGGCTGTGGCTAAT GCTCCTCAATATCTGCTTTTATGTGATTAATGGCTGTGGATAAAACCTTGATCCCAGGAAAGCAGGCCCAGCGGCT AAGCCTAGCCAAGAGCACCACTTTGGTTATGCTGGGAGCCCGAGTCATTTTGGCGATTTTCATTTTAACCAAGCTG TCATTTTAGTATGTGAGGAAAAAAAATAAAAGTGGTAGGAACTACACTCACAGAGAGCCATCAGGAAGGCACTG TGGAGGGGTAATCCGCGTAATGGCCCAGAACACGGCGTAACAGCTGGCTGTGGCTAATGCTCCTCAATATCTGCT TTTATGTGATTAATGGCTGTGGATAAAACCTTGATCCCAGGAAAGCAGGCCCAGCGGCTAAGCCTAGCCAAGAGC ACCACTTTGGTTATGCTGGGAGCCCGAGTCATTTTGGCGATTTTCATTTTAACCAAGCTGTCATTTTAGTATGTGAG GAAAAAAAATAAAAGTGGTAGGAACTACACTCACAGAGAGCGCTGCAAAGACCCATGACCCCATCTGCCTTTAA GAGGGGCAAGACAGGCACCCCTGAAATCAGAAATGACACAGAGAGCCCCACACACAGAGCAGCACAGAACAG ACGGGCCACCGGCCACAGGATCTTCAGAAGAAGGCTTGCTGTGTCGCTGCCAGATTAACGGGGCGAAAATGCGC ACTGGCAGCTCCAAGGAGAGAGCTGAGACCCACAGCCTGGGCCTGGATGCAAATTCTTCTCTGAGACTCAGGCG GTTGGCCAGGTGGGCACCGGGCCTCTGGGATCTGGATGCCACCCTCCAAGGGGCCTCAGATGCCAGCAGCAAGG GGCTGTCTGCCCTCGCAATGCGCCTTTCCAGACGCCCAAATCCTTCCCATTTGACCACCCTTTGCAGCCACCACTT TCTCAAAGTAGATTTACTTTCAATCTCCATTTGCTAAATCTCTTCCCTCTAAACCTTTTGGGGATGGAGGGATTAAG CCTGATTACAGATGTTGGGGGTGGTAGCAGGAGTCGGGCAGGAGGGGCCACCAAAAGCCAGCACATGCTCCTCC TAGGCTGAACCAGCCTCCGGCCGGCCTCGCCCCAGGATCTCGGCCCCCTCGCCCAGTGCGTGGGGCTGCCGCCG AGTGTGGGGCTGCCGCCGAGTGTGTGGCTCCGCTCCTGCCCACCCAGGGAGGGCAGCCCCTGTGCCACTGGCCT GGCCACCCGAGGGAGTCAGCAGGGTGGCTCACAGGCTCACTGAGAGAG |
| 27 | En4_4 00-P7 50(hT RPM1 ) | GCAAGAAAGAGGAGCTGCTTTTGGGGTCACAGAGGGGCCAGGGGAGGCAGAGCTTGTGCCCAACCTTGGGGTC AACCCGGCTGCCAATAGGCCCATGCAAACCCAAGACAGTGGGCCATCAGGAAGGCACTGTGGAGGGGTAATCCG CGTAATGGCCCAGAACACGGCGTAACAGCTGGCTGTGGCTAATGCTCCTCAATATCTGCTTTTATGTGATTAATGG CTGTGGATAAAACCTTGATCCCAGGAAAGCAGGCCCAGCGGCTAAGCCTAGCCAAGAGCACCACTTTGGTTATG CTGGGAGCCCGAGTCATTTTGGCGATTTTCATTTTAACCAAGCTGTCATTTTAGTATGTGAGGAAAAAAAATAAAA GTGGTAGGAACTACACTCACAGAGAGCGCTGCAAAGACCCATGACCCCATCTGCCTTTAAGAGGGGCAAGACAG GCACCCCTGAAATCAGAAATGACACAGAGAGCCCCACACACAGAGCAGCACAGAACAGACGGGCCACCGGCCA CAGGATCTTCAGAAGAAGGCTTGCTGTGTCGCTGCCAGATTAACGGGGCGAAAATGCGCACTGGCAGCTCCAAG GAGAGAGCTGAGACCCACAGCCTGGGCCTGGATGCAAATTCTTCTCTGAGACTCAGGCGGTTGGCCAGGTGGGC ACCGGGCCTCTGGGATCTGGATGCCACCCTCCAAGGGGCCTCAGATGCCAGCAGCAAGGGGCTGTCTGCCCTCG CAATGCGCCTTTCCAGACGCCCAAATCCTTCCCATTTGACCACCCTTTGCAGCCACCACTTTCTCAAAGTAGATTT ACTTTCAATCTCCATTTGCTAAATCTCTTCCCTCTAAACCTTTTGGGGATGGAGGGATTAAGCCTGATTACAGATGT TGGGGGTGGTAGCAGGAGTCGGGCAGGAGGGGCCACCAAAAGCCAGCACATGCTCCTCCTAGGCTGAACCAGC CTCCGGCCGGCCTCGCCCCAGGATCTCGGCCCCCTCGCCCAGTGCGTGGGGCTGCCGCCGAGTGTGGGGCTGCC GCCGAGTGTGTGGCTCCGCTCCTGCCCACCCAGGGAGGGCAGCCCCTGTGCCACTGGCCTGGCCACCCGAGGGA GTCAGCAGGGTGGCTCACAGGCTCACTGAGAGAG |
| 28 | En4_1 60-P7 50(hT RPM1 ) | CGGGGGGATGGAGGCCCCTCAGTCGGGAGGTGCAAGAAAGAGGAGCTGCTTTTGGGGTCACAGAGGGGCCAGG GGAGGCAGAGCTTGTGCCCAACCTTGGGGTCAGCTGGCTGTGGCTAATGCTCCTCAATATCTGCTTTTATGTGATT AATGGCTGTGGGCTGCAAAGACCCATGACCCCATCTGCCTTTAAGAGGGGCAAGACAGGCACCCCTGAAATCAG AAATGACACAGAGAGCCCCACACACAGAGCAGCACAGAACAGACGGGCCACCGGCCACAGGATCTTCAGAAG AAGGCTTGCTGTGTCGCTGCCAGATTAACGGGGCGAAAATGCGCACTGGCAGCTCCAAGGAGAGAGCTGAGAC CCACAGCCTGGGCCTGGATGCAAATTCTTCTCTGAGACTCAGGCGGTTGGCCAGGTGGGCACCGGGCCTCTGGG ATCTGGATGCCACCCTCCAAGGGGCCTCAGATGCCAGCAGCAAGGGGCTGTCTGCCCTCGCAATGCGCCTTTCCA GACGCCCAAATCCTTCCCATTTGACCACCCTTTGCAGCCACCACTTTCTCAAAGTAGATTTACTTTCAATCTCCATT TGCTAAATCTCTTCCCTCTAAACCTTTTGGGGATGGAGGGATTAAGCCTGATTACAGATGTTGGGGGTGGTAGCAG GAGTCGGGCAGGAGGGGCCACCAAAAGCCAGCACATGCTCCTCCTAGGCTGAACCAGCCTCCGGCCGGCCTCG CCCCAGGATCTCGGCCCCCTCGCCCAGTGCGTGGGGCTGCCGCCGAGTGTGGGGCTGCCGCCGAGTGTGTGGCT CCGCTCCTGCCCACCCAGGGAGGGCAGCCCCTGTGCCACTGGCCTGGCCACCCGAGGGAGTCAGCAGGGTGGC TCACAGGCTCACTGAGAGAG |
| 29 | En1_4 80-En 4 160 -P750 | TCCCCCAGAAAATGTAATTGGAAATATCAACTCATGTTGAATGTGTTTGCTGTACCTGTTTATGTTTTATTGATTATT TTCTGGGAGAAGTTTGGAGTTGTGTTTGGTTTGGCTCTGTGCAACCTCATTTATTTGCTCTGTAAAGCTGATAAGC ACAAGAAGTGAAATGACACTCAGGCATTTTAAGATGCGAGTCATCTTCATTCTGAACTAAGTGAAAACAAAACA GATTTTCTATCAACCTAAAATCCTCCTACTGGGGGCAGTAGATGTTTTTAGAAAATTGGCTTTGGCTTTGATTGACA ATGTTTGGAAGATGTCCTATAATTTGAGAGTGTATGTGTGTGTGTCTGGGTGTGTGTGTGTAAATGCTGGACCTGG GAAGATGGAAGCCCTCAGCCAGGAGAATAAGAGGACACAATACCCCCAAGCCTTCCATGACCTAGAGATTAAGG GGATTTAACGTCTTAAAACCTCCATCGGGGGGATGGAGGCCCCTCAGTCGGGAGGTGCAAGAAAGAGGAGCTGC TTTTGGGGTCACAGAGGGGCCAGGGGAGGCAGAGCTTGTGCCCAACCTTGGGGTCAGCTGGCTGTGGCTAATGC TCCTCAATATCTGCTTTTATGTGATTAATGGCTGTGGGCTGCAAAGACCCATGACCCCATCTGCCTTTAAGAGGGG CAAGACAGGCACCCCTGAAATCAGAAATGACACAGAGAGCCCCACACACAGAGCAGCACAGAACAGACGGGC CACCGGCCACAGGATCTTCAGAAGAAGGCTTGCTGTGTCGCTGCCAGATTAACGGGGCGAAAATGCGCACTGGC AGCTCCAAGGAGAGAGCTGAGACCCACAGCCTGGGCCTGGATGCAAATTCTTCTCTGAGACTCAGGCGGTTGGC CAGGTGGGCACCGGGCCTCTGGGATCTGGATGCCACCCTCCAAGGGGCCTCAGATGCCAGCAGCAAGGGGCTGT CTGCCCTCGCAATGCGCCTTTCCAGACGCCCAAATCCTTCCCATTTGACCACCCTTTGCAGCCACCACTTTCTCAA AGTAGATTTACTTTCAATCTCCATTTGCTAAATCTCTTCCCTCTAAACCTTTTGGGGATGGAGGGATTAAGCCTGAT TACAGATGTTGGGGGTGGTAGCAGGAGTCGGGCAGGAGGGGCCACCAAAAGCCAGCACATGCTCCTCCTAGGCT GAACCAGCCTCCGGCCGGCCTCGCCCCAGGATCTCGGCCCCCTCGCCCAGTGCGTGGGGCTGCCGCCGAGTGTG GGGCTGCCGCCGAGTGTGTGGCTCCGCTCCTGCCCACCCAGGGAGGGCAGCCCCTGTGCCACTGGCCTGGCCAC CCGAGGGAGTCAGCAGGGTGGCTCACAGGCTCACTGAGAGAG |
| 30 | En2_4 60-En 4_160 -P750( hTRP M1) | TGTGGGGCTCACACTGCATTTGCAGCTGGGTTCACCCTGACCTCAGGCCCAACTTAGATGAGGAAGGATTAGCAG TAATTAGTGCCATGTGCCGCCTTCTCCCAGCTCCCCGGGGCACGAACACTGCCCAGCTGATGAGGGGATTCTGAA AGAACCATTATGGGGATAAGTACTCTGTCAGCTGGTCTCAAAGAGGCTGAAACCCTCTGAGCTTCCCTCAGGGCC AGCTTCTAAATCCAGGTGCCAGGGATCTGCTCAGGAGCCCCGGCTCCAGGCCTCAACCTCTTGCCTGTCATTCCT TAGCATCTTACTTTGCTTCAGGGAAAAGGTGCACCTTCCTTAGAACCCCAGAAAATGCTGAAGTTCTAGGAGAGA CAGGGTTTATACTTGTAAGCCTTTATCAGCTGATATTTCACCTGTGAAAGCCTTGGGGAAGAGATTGGTTACACGG AACAGGATCCGGGGGGATGGAGGCCCCTCAGTCGGGAGGTGCAAGAAAGAGGAGCTGCTTTTGGGGTCACAGA GGGGCCAGGGGAGGCAGAGCTTGTGCCCAACCTTGGGGTCAGCTGGCTGTGGCTAATGCTCCTCAATATCTGCTT TTATGTGATTAATGGCTGTGGGCTGCAAAGACCCATGACCCCATCTGCCTTTAAGAGGGGCAAGACAGGCACCCC TGAAATCAGAAATGACACAGAGAGCCCCACACACAGAGCAGCACAGAACAGACGGGCCACCGGCCACAGGATC TTCAGAAGAAGGCTTGCTGTGTCGCTGCCAGATTAACGGGGCGAAAATGCGCACTGGCAGCTCCAAGGAGAGA GCTGAGACCCACAGCCTGGGCCTGGATGCAAATTCTTCTCTGAGACTCAGGCGGTTGGCCAGGTGGGCACCGGG CCTCTGGGATCTGGATGCCACCCTCCAAGGGGCCTCAGATGCCAGCAGCAAGGGGCTGTCTGCCCTCGCAATGC GCCTTTCCAGACGCCCAAATCCTTCCCATTTGACCACCCTTTGCAGCCACCACTTTCTCAAAGTAGATTTACTTTC AATCTCCATTTGCTAAATCTCTTCCCTCTAAACCTTTTGGGGATGGAGGGATTAAGCCTGATTACAGATGTTGGGG GTGGTAGCAGGAGTCGGGCAGGAGGGGCCACCAAAAGCCAGCACATGCTCCTCCTAGGCTGAACCAGCCTCCG GCCGGCCTCGCCCCAGGATCTCGGCCCCCTCGCCCAGTGCGTGGGGCTGCCGCCGAGTGTGGGGCTGCCGCCGA GTGTGTGGCTCCGCTCCTGCCCACCCAGGGAGGGCAGCCCCTGTGCCACTGGCCTGGCCACCCGAGGGAGTCAG CAGGGTGGCTCACAGGCTCACTGAGAGAG |
| 31 | En4_6 22-P1 061(h TRPM 1) | GAGCACTTGAGCTTTCAGGGTGCAGGGCCTGTGGGAAACGGGCCATGGACCTCCTCGGGGGGATGGAGGCCCCT CAGTCGGGAGGTGCAAGAAAGAGGAGCTGCTTTTGGGGTCACAGAGGGGCCAGGGGAGGCAGAGCTTGTGCCC AACCTTGGGGTCAACCCGGCTGCCAATAGGCCCATGCAAACCCAAGACAGTGGGCCATCAGGAAGGCACTGTGG AGGGGTAATCCGCGTAATGGCCCAGAACACGGCGTAACAGCTGGCTGTGGCTAATGCTCCTCAATATCTGCTTTTA TGTGATTAATGGCTGTGGATAAAACCTTGATCCCAGGAAAGCAGGCCCAGCGGCTAAGCCTAGCCAAGAGCACC ACTTTGGTTATGCTGGGAGCCCGAGTCATTTTGGCGATTTTCATTTTAACCAAGCTGTCATTTTAGTATGTGAGGAAA AAAAATAAAAGTGGTAGGAACTACACTCACAGAGAGCCCAGAGAGGGGGCAGCAGGGGGGTGGGGGCGAGC CTGGTGCTGGAAGTGTGAAAACAGCTGGGGGCCCAAGTGCCCTGACTCCCCTGGCCACCCACTCACTGCTTCCA CCCACTCTCACTTCCCTTGGGTTCACACTGTGTTGGTTGTAAGCCAGACCCCAAGAGGTGGAGGTCATGCCTCCA CCCTCCAAACTTCCACAGCCGACAGGCCTCCCAGAGGAGGTGGTGGGTCCTGGCCTCACAGCTATGAGGGCTCC ACAGCCTTCTATTATTTTATTTTCAGCTTGCTGGTCCTTAGATAAAAATCTATCTAAGACGATGGGAATACATGGCCT GTTCTCTCTGAGCTCCAGTCTGTTCCCTTGCATGTGGGAGGTGACATGCATGTGCCTGTAAGAGACAGAGGAGGG TCGGCAGCAAGGGTGGTACACTTCAGGCCAGGCTTGTGCTGCAAAGACCCATGACCCCATCTGCCTTTAAGAGG GGCAAGACAGGCACCCCTGAAATCAGAAATGACACAGAGAGCCCCACACACAGAGCAGCACAGAACAGACGG GCCACCGGCCACAGGATCTTCAGAAGAAGGCTTGCTGTGTCGCTGCCAGATTAACGGGGCGAAAATGCGCACTG GCAGCTCCAAGGAGAGAGCTGAGACCCACAGCCTGGGCCTGGATGCAAATTCTTCTCTGAGACTCAGGCGGTTG GCCAGGTGGGCACCGGGCCTCTGGGATCTGGATGCCACCCTCCAAGGGGCCTCAGATGCCAGCAGCAAGGGGCT GTCTGCCCTCGCAATGCGCCTTTCCAGACGCCCAAATCCTTCCCATTTGACCACCCTTTGCAGCCACCACTTTCTC AAAGTAGATTTACTTTCAATCTCCATTTGCTAAATCTCTTCCCTCTAAACCTTTTGGGGATGGAGGGATTAAGCCTG ATTACAGATGTTGGGGGTGGTAGCAGGAGTCGGGCAGGAGGGGCCACCAAAAGCCAGCACATGCTCCTCCTAGG CTGAACCAGCCTCCGGCCGGCCTCGCCCCAGGATCTCGGCCCCCTCGCCCAGTGCGTGGGGCTGCCGCCGAGTG TGGGGCTGCCGCCGAGTGTGTGGCTCCGCTCCTGCCCACCCAGGGAGGGCAGCCCCTGTGCCACTGGCCTGGCC ACCCGAGGGAGTCAGCAGGGTGGCTCACAGGCTCACTGAGAGAG |
| 32 | hGR M6(12 43) | GGGTCTCCAACTTGCCAACTGTAGATCTTGGAACCTTTCATCCTTCATAACTGCATAAGCCAATTCCTTCTAATAAA TCTGTATAATATATCTGTCTATATAATAAATATGTACTTACATAACTCTGTATGTTACATCTATCTATTCTATCTATCTAT CTATCTATCTATCTATCTATCTATCTATCATCTATCTATCTATTTATCTATTCTCTGTGTCTTTGGAGAACCCTGACATA GTAAGCAATCATATCACCTGCAAATGATGAAAGCTGTGTATTTTCCAAATCAGTCGTTTTATGTCTTTTTTTCTTGC ACTGACTAGTGCCCCCTAGAGGGAATGATAATTGGAATTATTGTCTTGCTCTGATTTTAAAGGAAGTAGATACTTC AAATAATTCATCATGGAGTGCAATATTTTCTGTAGGCTTTTAGTAGATAACTTCATCAGTTTAAAGAAGATCCTTAG ATTATGAAACATTTACAATTATGAATGAATATTAGATGTTATCAAATGCTTTTTCTGCATCCATTTAGATAATCATGTT TTTCCTTTAATCTGTTAATGCGGTGAATTACATTAATAGATTTCCTAAGTCATTAATCTGCTAAAGTGCATTTCTGGG ACAAACCAGACTTGGTTATGACATTGTATGTATTTCAGTTTGCAAATATTGGACTAGGATTTTTGTATCTATATTCCT TAGTTTGACCTGTAAATTTTATTTCTTGTACTAAGTATTAGCCTCACGAAAGGCATTGTCAAATGTTCAATTGATATA ATGCTAGCGGAGGGGTCTCCACCCTCGGAGCGGTCTCTCATCCCTCCCTAGAATCCTTAAATCCTCTCTCGCTCAG GGCCTCGGCCGCATCTGTCACAGACTTGTCCTGAACCGACAGCGGCTGGCGCAGGTGACTGGCTTGGGGCGGGA GCCTGGGTGTGCGCTGGGGATGGACCCCGAGGAAGAGGGGCCAAGCTGTCGGGAAGCGGCAGGGCTGGAGGG GTGGAGGCAGTGGTCGGGCGGGACCCCGGGCGACAGGGTTCGGCGCTTGTAAGAGCGAGACGGAGGCCCGGGC AGGCCGGCTGAGCTAACTCCCCAGAGCCGAAGTGGAAGGCGCGCCCCGAGCGCCTTCTCCCCAGGACCCCGGT GTCCCTCCCCGCGCCCCGAGCCCGCGCTCTCCTTCCCCCGCCCTCAGAGCGCTCCCCGCCCCTCTGTCTCCCCGC AGCCCGCTAGACGAGCCGA |
| 33 | mP14 88(mT RPM1 ) | TGTGCTGGGAAGATCCTCTTCCTAGTCTTCTAAGCCAGACTTCTAAAGGCTGTGGCTCTACCTTCTGAACCCCCAC AGCCAAGCAGTTCTCTTGGGGCAAGAAGCCACACCCAGGGAGGAGAAAGTTGGATCTTGGGTTACATGGTTGGC AAGTTAGCTTTTACTTACCTCTAGCCTTGTCTGTCTGTATTGTGTGCTCATTTTCTTTTTCTATGCCAGTGGGGCTCC TTCTCTATGTTCTGTGAGCCTTGTTTTTTCTCCTCTGTACATTAGCTGATACAAACACCCAAGGAAGTCATCTTGAG AGGGTGACACATAGGTGCACTAGAATAGACCTAGGAGAACTGCTGGATTGATTTGCTGTGTACCACTACCCTTCC CCTATTCCACACAGCAGCAGAGAAGACCTGTGTCCTGTCCTAGTCTGTGTTTTTAAGAGGGAAGACCATACCCCT GAAATATGATGGACAGATCCCTCACGTGGAACACAGAATGGATCTGAGATGGACACGGGGCACACACAACAAGG TATACTGTTTTGTTACCCAGTTCATGGGAGAAAGCATCCCCATTGGCTTCTAGGAGGGAGCTTCCATCTACCCAGT CTAGGCCTAAAAACTCAGCCTACTGATATATGAATGCCATGGGAAGCTGGCCTCAGTCTTTGCCAGTCAGATTTTG TCAACTTGACACAAGTGTTATTTGGGAAGAGAAACCTCAGTTGAGAAATTACCTCCCTCAGGTTGCCTGTAGGCA AGTCTGTAGGGTGTTTTCATGACTATTGATTGATGTGGGCAAGCTCAGCCCACAGTAGTGTTTGTGGAGTCACACT TGGGGGCAGGTGGTCCTGGGGTGTATAAGAAAGCAGGATGAGCAAGCTATGAGCAACCAAGCCTGTAAGCAGCA TCCCTCCATGGCTTCTGTACCTCTATGGCTTGAGTACCTGCCCTGACTTCCCTCAGTGATGACCTGAGAGTGGTAA GTTGAAATAAACCATTTCTTTCCCAACCTGCTTTTCATCATGGTATTTTATCTGCACCATAGAAACATTAAGACATA GCCCAACAAGGGGCCTTGGGCACCAGTGCTGAAGGTTCTCACATTTCACCTTGCTGGATCCCCAAATCCTCATCA TGTGCCCACCCCAAACCCTCTGCAGTCACCACTTTCTCAAAGTAGATTTTACTTCCAATTGCCATTTGCTAAATCT GCCTCCTAAAAACCTTGCGGGGGGAGTAGGGATTAAGGCTGATTACAGATGTTACAGAAGCTATGTAGTCTCTGA GCACTGGGTGGGAGTCCAGCCGGAGGGTGAGAAGCAGTCCAGCTCCTTCTGAGCTGACTGAGCCATCAATATCA TCTCAAACTCTGAGCCCTGCTGTCCAGTCTCATAAAGAGGACTCTCTTACCTCAGCTGACCAGGGCCTCCCATGC AGCAGGCTGGACCTCACAGGGAATGCAGAAGCACCCATGGGGTCCATGAGGAAG |
| 34 | mP52 8(mT RPM1 ) | AGTGATGACCTGAGAGTGGTAAGTTGAAATAAACCATTTCTTTCCCAACCTGCTTTTCATCATGGTATTTTATCTGC ACCATAGAAACATTAAGACATAGCCCAACAAGGGGCCTTGGGCACCAGTGCTGAAGGTTCTCACATTTCACCTTG CTGGATCCCCAAATCCTCATCATGTGCCCACCCCAAACCCTCTGCAGTCACCACTTTCTCAAAGTAGATTTTACTT CCAATTGCCATTTGCTAAATCTGCCTCCTAAAAACCTTGCGGGGGGAGTAGGGATTAAGGCTGATTACAGATGTTA CAGAAGCTATGTAGTCTCTGAGCACTGGGTGGGAGTCCAGCCGGAGGGTGAGAAGCAGTCCAGCTCCTTCTGAG CTGACTGAGCCATCAATATCATCTCAAACTCTGAGCCCTGCTGTCCAGTCTCATAAAGAGGACTCTCTTACCTCAG CTGACCAGGGCCTCCCATGCAGCAGGCTGGACCTCACAGGGAATGCAGAAGCACCCATGGGGTCCATGAGGAA G |
| 35 | mEn1 _984( mTRP M1) | TTTCTGATCAGAACTGAGGCCTCTTTCAGGGGAGGGAACTCATGCCTGGTACTGCACAACCGCTCAAAAGCCAAT GACTCAGGTAGTCAGAGGGCCCAAGGGGAACCTACTGCTGTTGTTTTGCTAAATGAACACGTTAAACTGCCTTAT ATTTATGTTTGTACTCGTAGATCAATGCTGCCTTCCATCTTGACCAGAGAAGCCACTTTCTGCTGGGGCAGCAATAT AGACTCATAACTGCTCCGGGTGCTGAGAATAAGAGATTTTGAGATTGCCCCGCCCTGACTGGGACATCCCTGCTG AAATGGAAAGAACTAGAACTGATTTGTCTAGCATGGCAGAAACATGGCTGAGCCCTTGCGTATGGTCTCAGCTGA GATATACTGAGGACAGGGTAAACCACCACACAGCCATTCACCATGGTCAGGTGTGCCCCAGAGTCTGCAAGATTT CAAAAACCCGAGAATCCTGACATGAAGTATCTCATAAATAAGCTTTTAGGTGTTGACATGTTTATTGAGTTCAAAA CCTATAGAATTGAAGTTGACTTCATTTATTCATTTTTTGTAAGTGGCATTAATCATAGTGGCAGTCCATAGCTGAAC CTGCTCTCCCCCCCCCCCCCCCCCCGCTTGAGACAGTCTCACCATGTAGTTTAGATGATCATGAACCCTGACCCAG CCTTTCAAGTTTTGGCATTGCAGGTGTGTGCAAACACCAGGCAGGCTATTCTTTCTAACTGAGTTTTTAGACATTG AATTCCTTTAGGTTGCAGAAGATATTGGTGAATAGGGATCCACTTACTCCTAGCTGGGGACTTCCAGTTTCTAGAT CCAGGCCCAGACTCTAACTTCAATTTCTCTCTCTCTCTCTCTCTCTCTCTCTCTCTCTCTCTCTCTCTCTCTCTCT CTCTCTCTCTCTCCATTTGTCTCTTTCTCTCTGTCTCTGCTCTGTTTCTATCTCTATCTCTCTGTGTGTCTCCAG |
| 36 | mEn1 _664( mTRP M1) | AACCGCTCAAAAGCCAATGACTCAGGTAGTCAGAGGGCCCAAGGGGAACCTACTGCTGTTGTTTTGCTAAATGA ACACGTTAAACTGCCTTATATTTATGTTTGTACTCGTAGATCAATGCTGCCTTCCATCTTGACCAGAGAAGCCACTT TCTGCTGGGGCAGCAATATAGACTCATAACTGCTCCGGGTGCTGAGAATAAGAGATTTTGAGATTGCCCCGCCCT GACTGGGACATCCCTGCTGAAATGGAAAGAACTAGAACTGATTTGTCTAGCATGGCAGAAACATGGCTGAGCCC TTGCGTATGGTCTCAGCTGAGATATACTGAGGACAGGGTAAACCACCACACAGCCATTCACCATGGTCAGGTGTG CCCCAGAGTCTGCAAGATTTCAAAAACCCGAGAATCCTGACATGAAGTATCTCATAAATAAGCTTTTAGGTGTTG ACATGTTTATTGAGTTCAAAACCTATAGAATTGAAGTTGACTTCATTTATTCATTTTTTGTAAGTGGCATTAATCATA GTGGCAGTCCATAGCTGAACCTGCTCTCCCCCCCCCCCCCCCCCCGCTTGAGACAGTCTCACCATGTAGTTTAGAT GATCATGAACCCTGACCCAGCCTTTCAAGTTTTGGCATTGCAGGTGTGTGCAAACACCAG |
| 37 | mEn2 _1187 (mTR PM1) | AGATACTAGAAGAGGGAGATGCTGCCCAGCTCTGAAGCTGGGGTTTCCCAGCAAATCCCAGGCATGCGCATGGA GAAGCTTTGCCCAGGGAAGCTCAGAGTATCGAGCCAGTTCTCAACCCTACAGACCTTCTTCTCTGCCAATCAACC AGTCAGGCCTCTGGGAACCCAGGCTCAGTCTGGATCTAGTACCCAGTCCCAGAAGGCCCTGAAGCCACGTTTGC AGAGAGGAGCAAGGGGCTGAAAGAGCCAAACTCTGTCACTGAACTTACCTGCCTCAGCTGCTGAGCAAGCGGG AAGGGTGCAGCCTAGCCTCCTTCAGCCTCCTCCTGGGCCCAGGCAGGAAGCTGAATGAGGGGGGCTCACTCTTT AAGAGGAGCAGCCCACATGAAGAGCATGTGGGTGCCTATGCCCGGTCCTCATGATCCCCGTGAGACAGAGGCAG CCTCGTGACCTGGCTCCCAGGCTGCTCAGCGTGGTATGCACAAAAGCCACCTCCCTTTGAAGTGGTCCCCACCTC CCCTGCCCCAGCCAGGCCTTTCAGTGCTGCAAATGTCAGCCCACTTTGCATAATTGAGACAATTGGGCATAATCAT TCTTTCAGAAGCCCCCACTGGCAGGCAGCTTATGCCCCAGCCCTCATGAGCCAGGAAGAGGAGCCACGTGCTTC CAATTACTGCGGAGCCTTTTTAAGTTGGTCAGAGTTGGGCCAGAGGTTGCGACGACTCCGACGGCAAACGCTGG GTGAGTCCCACATGACTTTCAGGCCCACGTGAAGCTTCTGATCCCATTCTGTTTCCACTCCACCAGCAATCTGCCT GACTGGGGTCACAGTGGCCACCACAGTGAGAGCTTTGTGGATTTGCTGTACCAACACACCTGCATGTGTCCTAGC CTCTGGCATGCTTTCTCTGCCTGCTAGAAGGAAAGTCCAGAAAGGCCCCTTGATATGTATCCCTTTGGACTTTTCT TCAGCACAGGTGGTAGTGGGGGCTCCTGGGAGCAGACCCCTCTCTGCTTTACTAGGGGCTTGTATACCTGTAGAT GGGTGGGATGCCTGGACAGGTTCCCATCTGCACAAGGGGACACCGAGGGTAGGGAGTGGCTGACTTGCCCAGA GAGTGTGGGAACCGGAGCAGGCCCGGCTGCACACTTTAGGATGTTCTGTTCACTATAGAGCCCTGAGGA |
| 38 | mEn2 _542( mTRP M1) | GAAGGCCCTGAAGCCACGTTTGCAGAGAGGAGCAAGGGGCTGAAAGAGCCAAACTCTGTCACTGAACTTACCT GCCTCAGCTGCTGAGCAAGCGGGAAGGGTGCAGCCTAGCCTCCTTCAGCCTCCTCCTGGGCCCAGGCAGGAAGC TGAATGAGGGGGGCTCACTCTTTAAGAGGAGCAGCCCACATGAAGAGCATGTGGGTGCCTATGCCCGGTCCTCAT GATCCCCGTGAGACAGAGGCAGCCTCGTGACCTGGCTCCCAGGCTGCTCAGCGTGGTATGCACAAAAGCCACCT CCCTTTGAAGTGGTCCCCACCTCCCCTGCCCCAGCCAGGCCTTTCAGTGCTGCAAATGTCAGCCCACTTTGCATA ATTGAGACAATTGGGCATAATCATTCTTTCAGAAGCCCCCACTGGCAGGCAGCTTATGCCCCAGCCCTCATGAGCC AGGAAGAGGAGCCACGTGCTTCCAATTACTGCGGAGCCTTTTTAAGTTGGTCAGAGTTGGGCCAGAGGTTGCGA CGACTCCGACGGCAAACGCTG |
| 39 | mEn4 _876( mTRP M1) | ATCTAAGCCCCAGGGATGTGGAGTTGGGGCCAGTGAGCTAGGTTCCTAGGAGTAAGAACCCTTTGTCCCTGGCTG CTTTTACATCTTCGACACCAGGCTTGGCTCCCCCGTCTCCCCTTGTCTCTCTGAGCTGCCCATGAGCTCCATTCTC ACCTCTTCACGGAAATGACTCCTGACTCCCAGCATCACAAAGTGATGGGAGCTGGGTTCATCGGCAGGGCCTGCT TTTCCGGGATCAAAGTCTTATCGACAGCCTTTAATCACATGAAAGCAGATATTCCCAGGGCATTAGGCCCCACCAG ATGCTGTGCGCCTGCCTGGGCCATTAGGAGATTATGCTTGGACCCAGCCAGCACTCGGCACCCATGGCTTGGGGA TGTGGGACTCTTGATCGTTAAGCCGAGGACCCAGCACTCTGGGTACCTGTGCTTTCTCCTGCCCTCTCTGTAATGG CCCCTAGACTTCTTTGCTTAGGAATCTCCTCTCCCCAAGCAGATGTGATGATCCCTTCGCATGGCTCTGCTGACCT CAGAGCACATCCTCCTGGCTTGATGCTCAGTCATGCTGGCAGCCTGGTAAAGCAGGGAACCTACCTTCCCCCATG GGCTGGAGGCTCTGTAGGCCCAAGGCAAGTGGGTGTCCCTGTAGGTGCCAGAGTAGAAAAGGCTGAGCCCGAAT CTGGAGGAACAGGTATTCAGCAGGTACCAGGGCTATGGAGAATTGAAAAGGCCTTGGAGGCCAGAAGCCATAGC ACATGTCACTCTGCATGGTGAGCTGACCACTCAAAGCAGACTTAACCTAGGATGCACCACTGTCCAGCCTAACGT GACCTCATGGCAGGGGCTTTATTCTATATTTCTATGTATATTTCATATA |
| 40 | mEn1 _664-mP52 8(mT RPM1 ) | AACCGCTCAAAAGCCAATGACTCAGGTAGTCAGAGGGCCCAAGGGGAACCTACTGCTGTTGTTTTGCTAAATGA ACACGTTAAACTGCCTTATATTTATGTTTGTACTCGTAGATCAATGCTGCCTTCCATCTTGACCAGAGAAGCCACTT TCTGCTGGGGCAGCAATATAGACTCATAACTGCTCCGGGTGCTGAGAATAAGAGATTTTGAGATTGCCCCGCCCT GACTGGGACATCCCTGCTGAAATGGAAAGAACTAGAACTGATTTGTCTAGCATGGCAGAAACATGGCTGAGCCC TTGCGTATGGTCTCAGCTGAGATATACTGAGGACAGGGTAAACCACCACACAGCCATTCACCATGGTCAGGTGTG CCCCAGAGTCTGCAAGATTTCAAAAACCCGAGAATCCTGACATGAAGTATCTCATAAATAAGCTTTTAGGTGTTG ACATGTTTATTGAGTTCAAAACCTATAGAATTGAAGTTGACTTCATTTATTCATTTTTTGTAAGTGGCATTAATCATA GTGGCAGTCCATAGCTGAACCTGCTCTCCCCCCCCCCCCCCCCCCGCTTGAGACAGTCTCACCATGTAGTTTAGAT GATCATGAACCCTGAGTGATGACCTGAGAGTGGTAAGTTGAAATAAACCATTTCTTTCCCAACCTGCTTTTCATCA TGGTATTTTATCTGCACCATAGAAACATTAAGACATAGCCCAACAAGGGGCCTTGGGCACCAGTGCTGAAGGTTC TCACATTTCACCTTGCTGGATCCCCAAATCCTCATCATGTGCCCACCCCAAACCCTCTGCAGTCACCACTTTCTCA AAGTAGATTTTACTTCCAATTGCCATTTGCTAAATCTGCCTCCTAAAAACCTTGCGGGGGGAGTAGGGATTAAGGC TGATTACAGATGTTACAGAAGCTATGTAGTCTCTGAGCACTGGGTGGGAGTCCAGCCGGAGGGTGAGAAGCAGT CCAGCTCCTTCTGAGCTGACTGAGCCATCAATATCATCTCAAACTCTGAGCCCTGCTGTCCAGTCTCATAAAGAGG ACTCTCTTACCTCAGCTGACCAGGGCCTCCCATGCAGCAGGCTGGACCTCACAGGGAATGCAGAAGCACCCATG GGGTCCATGAGGAAG |
| 41 | mEn2 542-mP52 8(mT RPM1 ) | GAAGGCCCTGAAGCCACGTTTGCAGAGAGGAGCAAGGGGCTGAAAGAGCCAAACTCTGTCACTGAACTTACCT GCCTCAGCTGCTGAGCAAGCGGGAAGGGTGCAGCCTAGCCTCCTTCAGCCTCCTCCTGGGCCCAGGCAGGAAGC TGAATGAGGGGGGCTCACTCTTTAAGAGGAGCAGCCCACATGAAGAGCATGTGGGTGCCTATGCCCGGTCCTCAT GATCCCCGTGAGACAGAGGCAGCCTCGTGACCTGGCTCCCAGGCTGCTCAGCGTGGTATGCACAAAAGCCACCT CCCTTTGAAGTGGTCCCCACCTCCCCTGCCCCAGCCAGGCCTTTCAGTGCTGCAAATGTCAGCCCACTTTGCATA ATTGAGACAATTGGGCATAATCATTCTTTCAGAAGCCCCCACTGGCAGGCAGCTTATGCCCCAGCCCTCATGAGCC AGGAAGAGGAGCCACGTGCTTCCAATTACTGCGGAGCCTTTTTAAGTTGGTCAGAGTTGGGCCAGAGGTTGCGA CGACTCCGACGGCAAACGCTGAGTGATGACCTGAGAGTGGTAAGTTGAAATAAACCATTTCTTTCCCAACCTGCT TTTCATCATGGTATTTTATCTGCACCATAGAAACATTAAGACATAGCCCAACAAGGGGCCTTGGGCACCAGTGCTG AAGGTTCTCACATTTCACCTTGCTGGATCCCCAAATCCTCATCATGTGCCCACCCCAAACCCTCTGCAGTCACCAC TTTCTCAAAGTAGATTTTACTTCCAATTGCCATTTGCTAAATCTGCCTCCTAAAAACCTTGCGGGGGGAGTAGGGA TTAAGGCTGATTACAGATGTTACAGAAGCTATGTAGTCTCTGAGCACTGGGTGGGAGTCCAGCCGGAGGGTGAGA AGCAGTCCAGCTCCTTCTGAGCTGACTGAGCCATCAATATCATCTCAAACTCTGAGCCCTGCTGTCCAGTCTCATA AAGAGGACTCTCTTACCTCAGCTGACCAGGGCCTCCCATGCAGCAGGCTGGACCTCACAGGGAATGCAGAAGCA CCCATGGGGTCCATGAGGAAG |
| 42 | mEn4 _876-mP52 8(mT RPM1 ) | GAAGGCCCTGAAGCCACGTTTGCAGAGAGGAGCAAGGGGCTGAAAGAGCCAAACTCTGTCACTGAACTTACCT GCCTCAGCTGCTGAGCAAGCGGGAAGGGTGCAGCCTAGCCTCCTTCAGCCTCCTCCTGGGCCCAGGCAGGAAGC TGAATGAGGGGGGCTCACTCTTTAAGAGGAGCAGCCCACATGAAGAGCATGTGGGTGCCTATGCCCGGTCCTCAT GATCCCCGTGAGACAGAGGCAGCCTCGTGACCTGGCTCCCAGGCTGCTCAGCGTGGTATGCACAAAAGCCACCT CCCTTTGAAGTGGTCCCCACCTCCCCTGCCCCAGCCAGGCCTTTCAGTGCTGCAAATGTCAGCCCACTTTGCATA ATTGAGACAATTGGGCATAATCATTCTTTCAGAAGCCCCCACTGGCAGGCAGCTTATGCCCCAGCCCTCATGAGCC AGGAAGAGGAGCCACGTGCTTCCAATTACTGCGGAGCCTTTTTAAGTTGGTCAGAGTTGGGCCAGAGGTTGCGA CGACTCCGACGGCAAACGCTGAGTGATGACCTGAGAGTGGTAAGTTGAAATAAACCATTTCTTTCCCAACCTGCT TTTCATCATGGTATTTTATCTGCACCATAGAAACATTAAGACATAGCCCAACAAGGGGCCTTGGGCACCAGTGCTG AAGGTTCTCACATTTCACCTTGCTGGATCCCCAAATCCTCATCATGTGCCCACCCCAAACCCTCTGCAGTCACCAC TTTCTCAAAGTAGATTTTACTTCCAATTGCCATTTGCTAAATCTGCCTCCTAAAAACCTTGCGGGGGGAGTAGGGA TTAAGGCTGATTACAGATGTTACAGAAGCTATGTAGTCTCTGAGCACTGGGTGGGAGTCCAGCCGGAGGGTGAGA AGCAGTCCAGCTCCTTCTGAGCTGACTGAGCCATCAATATCATCTCAAACTCTGAGCCCTGCTGTCCAGTCTCATA AAGAGGACTCTCTTACCTCAGCTGACCAGGGCCTCCCATGCAGCAGGCTGGACCTCACAGGGAATGCAGAAGCA CCCATGGGGTCCATGAGGAAG |
| 43 | GFP-Nano Luc | ATGGTGAGCAAGGGCGAGGAGCTGTTCACCGGGGTGGTGCCCATCCTGGTCGAGCTGGACGGCGACGTAAACG GCCACAAGTTCAGCGTGTCCGGCGAGGGCGAGGGCGATGCCACCTACGGCAAGCTGACCCTGAAGTTCATCTGC ACCACCGGCAAGCTGCCCGTGCCCTGGCCCACCCTCGTGACCACCCTGACCTACGGCGTGCAGTGCTTCAGCCG CTACCCCGACCACATGAAGCAGCACGACTTCTTCAAGTCCGCCATGCCCGAAGGCTACGTCCAGGAGCGCACCA TCTTCTTCAAGGACGACGGCAACTACAAGACCCGCGCCGAGGTGAAGTTCGAGGGCGACACCCTGGTGAACCG CATCGAGCTGAAGGGCATCGACTTCAAGGAGGACGGCAACATCCTGGGGCACAAGCTGGAGTACAACTACAAC AGCCACAACGTCTATATCATGGCCGACAAGCAGAAGAACGGCATCAAGGTGAACTTCAAGATCCGCCACAACAT CGAGGACGGCAGCGTGCAGCTCGCCGACCACTACCAGCAGAACACCCCCATCGGCGACGGCCCCGTGCTGCTGC CCGACAACCACTACCTGAGCACCCAGTCCGCCCTGAGCAAAGACCCCAACGAGAAGCGCGATCACATGGTCCTG CTGGAGTTCGTGACCGCCGCCGGGATCACTCTCGGCATGGACGAGCTGTACAAGGATATCTCCGGAGGTATGGTC TTCACACTCGAAGATTTCGTTGGGGACTGGCGACAGACAGCCGGCTACAACCTGGACCAAGTCCTTGAACAGGG AGGTGTGTCCAGTTTGTTTCAGAATCTCGGGGTGTCCGTAACTCCGATCCAAAGGATTGTCCTGAGCGGTGAAAA TGGGCTGAAGATCGACATCCATGTCATCATCCCGTATGAAGGTCTGAGCGGCGACCAAATGGGCCAGATCGAAAA AATTTTTAAGGTGGTGTACCCTGTGGATGATCATCACTTTAAGGTGATCCTGCACTATGGCACACTGGTAATCGAC GGGGTTACGCCGAACATGATCGACTATTTCGGACGGCCGTATGAAGGCATCGCCGTGTTCGACGGCAAAAAGATC ACTGTAACAGGGACCCTGTGGAACGGCAACAAAATTATCGACGAGCGCCTGATCAACCCCGACGGCTCCCTGCT GTTCCGAGTAACCATCAACGGAGTGACCGGCTGGCGGCTGTGCGAACGCATTCTGGCGTAA |
| 44 | GFP-Nano Luc ( ami no acid seque nce) | MVSKGEELFTGVVPILVELDGDVNGHKFSVSGEGEGDATYGKLTLKFICTTGKLPVPWPTLVTTLTYGVQCFSRYPDH MKQHDFFKSAMPEGYVQERTIFFKDDGNYKTRAEVKFEGDTLVNRIELKGIDFKEDGNILGHKLEYNYNSHNVYIM ADKQKNGIKVNFKIRHNIEDGSVQLADHYQQNTPIGDGPVLLPDNHYLSTQSALSKDPNEKRDHMVLLEFVTAAGIT LGMDELYKDISGGMVFTLEDFVGDWRQTAGYNLDQVLEQGGVSSLFQNLGVSVTPIQRIVLSGENGLKIDIHVIIPYE GLSGDQMGQIEKIFKVVYPVDDHHFKVILHYGTLVIDGVTPNMIDYFGRPYEGIAVFDGKKITVTGTLWNGNKIIDER LINPDGSLLFRVTINGVTGWRLCERILA |
| 45 | Kozak | GCCACC |
| 46 | PsCat Ch2.0 | ATGCGACCCCAAATACTCCTCTTGCTGGCTTTGTTGACCCTTGGACTGGCTAACGGAACAGAAGGTCCCAACTTC TACGTTCCTTTCAGCAATAAGACAGGCGTAGTCAGATCCATGGGCTTCCAGCTCAATCCGGAATACCTGAACGAA ACAATTTTGCTGGACGACTGTACGCCAATCTATCTGAACGTCGGTCCCCTGTGGGAGCAAAAGGTTGCCCGAGGT ACGCAATGGTTTGGTGTTATCCTCTCGTTGGCGTTTCTCATCTATTATATATGGATAACATATAAGGCGACTTGCGGA TGGGAAGAACTGTACGTGTGTACGATCGAATTTTGTAAAATTGTAATTGAACTCTACTTCGAGTTTTCGCCTCCGG CGATGATATATCAGACCAACGGAGAGGTAACGCCCTGGTTGCGATATGCTGAATGGCTCTTGACCTGCCCAGTCAT TTGCATACATTTGAGTAACATTACAGGACTCAATGACGACTATTCGGGCCGAACTATGTCCCTGATCACATCCGAC CTGGGAGGCATCTGCATGGCCGTTACTTCCGCCTTGAGTAAAGGCTGGTTGAAGTGGCTCTTTTTCGTGATCGGC TGTTGTTACGGTGCTAGTACGTTTTATCATGCGGCGCTCATATATATAGAAAGTTACTACACTATGCCTCACGGTGTT TGTAAAAACATGGTATTGGCGATGGCAGCGGTATTTTTTACAAGTTGGTTCATGTTTCCCGGTTTGTTCCTGGCAG GCCCTGAAGGAACCAATGCACTGTCGTGGGCGGGCTCGACTATCGGTCATACCGTGGCAGATTTGCTCTCCAAAA ACGCCTGGGGTATGATTGGTCACTTCCTCCGACTGGAGATACACAAACATATAATTATTCATGGTGATGTTCGTCGC CCGATAACGGTTAATACCTTGGGACGAGAAGTCACGGTGTCGTGTTTTGTCGATAAAGAAGAGGAGGATGAAGA CGAACGTATCAGCACTAAGACCTACGCGAACCGGGCACTCGAGAAATCCAGAATTACTTCTGAAGGGGAGTATAT CCCTCTGGATCAAATAGACATCAATGTTGTCGACACTAGTTCTAGAtCTAGATTTTGCTATGAAAATGAAGTTTAA |
| 47 | PsCat Ch2.0 | MRPQILLLLALLTLGLANGTEGPNFYVPFSNKTGVVRSMGFQLNPEYLNETILLDDCTPIYLNVGPLWEQKVARGTQ WFGVILSLAFLIYYIWITYKATCGWEELYVCTIEFCKIVIELYFEFSPPAMIYQTNGEVTPWLRYAEWLLTCPVICIHLSN ITGLNDDYSGRTMSLITSDLGGICMAVTSALSKGWLKWLFFVIGCCYGASTFYHAALIYIESYYTMPHGVCKNMVLA MAAVFFTSWFMFPGLFLAGPEGTNALSWAGSTIGHTVADLLSKNAWGMIGHFLRLEIHKHIIIHGDVRRPITVNTLGR EVTVSCFVDKEEEDEDERISTKTYANRALEKSRITSEGEYIPLDQIDINVVDTSSRSRFCYENEV |

The above description is merely a preferred example of the present application and does not constitute any limitation on the present application. Any person skilled in the art may utilize the technical content disclosed above to change or modify the present application into equivalent examples with equivalent variations. However, any simple changes, equivalent variations, and modifications to the above examples based on the technical essence of the present application that do not depart from the technical solution of the present application shall still fall within the scope of protection of the technical solution of the present application.

## Claims

1. An enhancer or enhancer variant, wherein the enhancer comprises the nucleotide sequence as shown in SEQ ID NO: 4, SEQ ID NO: 35, SEQ ID NO: 6, SEQ ID NO: 37, SEQ ID NO: 9, SEQ ID NO: 10 or SEQ ID NO: 39, or a nucleotide sequence truncated from the 5'-end or 3'-end of the nucleotide sequence as shown in SEQ ID NO: 4, SEQ ID NO: 35, SEQ ID NO: 6, SEQ ID NO: 37, SEQ ID NO: 9, SEQ ID NO: 10 or SEQ ID NO: 39 to 40-910 base pairs in length, preferably to 56-880 base pairs in length;
wherein the enhancer variant is at least 60%, 65%, 70%, 75%, 80%, 85%, 90%, 95%, 96%, 97%, 98%, or 99% identical to the enhancer.

2. The enhancer or enhancer variant according to claim 1, wherein the enhancer comprises the nucleotide sequence as shown in any one of SEQ ID NOs: 4-14 and 35-39, or a nucleotide sequence having at least 60%, 65%, 70%, 75%, 80%, 85%, 90%, 95%, 96%, 97%, 98%, or 99% sequence identity to any one of SEQ ID NOs: 4-14 and 35-39.

3. A promoter or promoter variant, wherein the promoter comprises the nucleotide sequence as shown in SEQ ID NO: 1 or SEQ ID NO: 33, or a nucleotide sequence truncated from the 5'-end or 3'-end of the nucleotide sequence as shown in SEQ ID NO: 1 or SEQ ID NO: 33 to 500-800 base pairs in length, preferably to 550-750 base pairs in length;
wherein the promoter variant is at least 60%, 65%, 70%, 75%, 80%, 85%, 90%, 95%, 96%, 97%, 98%, or 99% identical to the promoter.

4. The promoter or promoter variant according to claim 3, wherein the promoter comprises the nucleotide sequence as shown in any one of SEQ ID NOs: 1-3 and 33-34, or a nucleotide sequence having at least 60%, 65%, 70%, 75%, 80%, 85%, 90%, 95%, 96%, 97%, 98%, or 99% sequence identity to any one of SEQ ID NOs: 1-3 and 33-34.

5. An isolated nucleic acid molecule, comprising an enhancer and a promoter, wherein:
the enhancer is the enhancer according to claim 1 or 2; and/or
the promoter is the promoter according to claim 3 or 4.

6. An isolated nucleic acid molecule according to claim 5, wherein the isolated nucleic acid molecule consists of: one or more of the enhancers or enhancer variants, one or more of the promoters or promoter variants, and optionally a spacer separating the enhancer or enhancer variant from the promoter or promoter variant.

7. The isolated nucleic acid molecule according to claim 6, wherein the isolated nucleic acid molecule comprises or consists of a sequence selected from the group consisting of SEQ ID NOs: 15-31 and 40-42, or comprises or consists of a sequence having at least 60% identity to the sequence selected from the group consisting of SEQ ID NOs: 15-31 and 40-42.

8. A nucleic acid expression vector, comprising the isolated nucleic acid molecule according to any one of claims 5 to 7.

9. The nucleic acid expression vector according to claim 8, wherein the nucleic acid expression vector is a recombinant adeno-associated virus vector (AAV), in particular wherein the nucleic acid expression vector is a recombinant AAV1, AAV2, AAV3, AAV4, AAV5, AAV6, AAV7, AAV8, AAV9, AAV10, AAV11 or AAV12 vector, or a derivative variant of the above recombinant vector.

10. The nucleic acid expression vector according to any one of claims 8 to 9, further comprising:
a transgene, wherein the transgene is a photosensitive protein gene, in particular a photosensitive protein gene comprising or consisting of SEQ ID NO: 46 or 47, and
b. a sequence encoding capsid protein.

11. An adeno-associated virus particle, virus-like particle, or non-viral particle, comprising the isolated nucleic acid molecule according to any one of claims 5 to 7 or the nucleic acid expression vector according to any one of claims 8 to 10.

12. A pharmaceutical agent, wherein the pharmaceutical agent is selected from the group consisting of the isolated nucleic acid molecule according to any one of claims 5 to 7, the nucleic acid expression vector according to any one of claims 8 to 10, and the adeno-associated virus particle, virus-like particle, or non-viral particle according to claim 11, and used as a medicament.

13. A pharmaceutical agent, wherein the pharmaceutical agent is selected from the group consisting of the isolated nucleic acid molecule according to any one of claims 5 to 7, the nucleic acid expression vector according to any one of claims 8 to 10, and the adeno-associated virus particle, virus-like particle, or non-viral particle according to claim 11, and used for treating congenital stationary night blindness (CSBN1) or rod-cone and cone-rod dystrophy, more preferably retinitis pigmentosa and macular degeneration, or other diseases for which the therapeutic effects are achieved by expressing therapeutic genes in bipolar cells.

14. A pharmaceutical agent, wherein the pharmaceutical agent is selected from the group consisting of the isolated nucleic acid molecule according to any one of claims 5 to 7, the nucleic acid expression vector according to any one of claims 8 to 10, and the adeno-associated virus particle, virus-like particle, or non-viral particle according to claim 11, and wherein the pharmaceutical agent is administered by:
a. intravitreal injection,
b. subretinal injection,
c. choroidal injection, or
d. eye drops.
